# EUROPÄISCHE PATENTSCHRIFT

(11) **EP 2 394 703 B1**
(45) Veröffentlichungstag und Bekanntmachung des Hinweises auf die Patenterteilung: **23.12.2015**
(21) Anmeldenummer: 10165901.9
(22) Anmeldetag: 14.06.2010
(51) Int. Cl.: A61Q 5/00, A61K 8/42, A61Q 19/00, A61K 8/34

(54) **Kühlmischungen mit verstärkter Kühlwirkung von 5-Methyl-2-(propan-2-yl)cyclohexyl-N-ethyloxamat**
Cooling mixture with reinforced cooling effect of 5-methyl-2-(propan-2-yl)cyclohexyl-N-ethyloxamate
Mélanges froids dotés d'un effet refroidissant renforcé de 5-méthyle-2-(propane-2-yl)cyclohexyl-N-éthyloxamate

(43) Veröffentlichungstag der Anmeldung: 14.12.2011
(73) Patentinhaber: Symrise AG, 37603 Holzminden (DE)
(72) Erfinder: Küper, Thomas, 48734, Reken (DE); Oertling, Heiko, 37603, Holzminden (DE); Lange, Sabine, 37603 Holzminden (DE)
(74) Vertreter: Fabry, Bernd

(56) Entgegenhaltungen:
- EP-A2- 2 033 688
- WATSON H R ET AL: "NEW COMPOUNDS WITH THE MENTHOL COOLING EFFECT", JOURNAL OF THE SOCIETY COSMETIC CHEMISTS, SOCIETY OF COSMETIC CHEMISTS, US, Bd. 29, Nr. 4, 1. Januar 1978 (1978-01-01) , Seiten 185-200, XP009045124, ISSN: 0037-9832
- ERMAN M B: "COOLING AGENTS AND SKIN CARE APPLICATIONS", COSMETICS & TOILETRIES, WHEATON, IL, US, Bd. 120, Nr. 5, 1. Mai 2005 (2005-05-01), Seiten 105-118, XP009051809, ISSN: 0361-4387

## Beschreibung

Die Erfindung betrifft eine Kühlmischung umfassend oder bestehend aus 5-Methyl-2-(propan-2-yl)cyclohexyl-N-ethyloxamat und ein, zwei, drei oder mehreren bestimmten Polyolen. Sie betrifft ferner eine kosmetische Zubereitung, umfassend eine solche Kühlmischung oder einen Hygieneartikel, umfassend eine solche Kühlmischung. Die Erfindung betrifft außerdem die Verwendung bestimmter Polyole zur Verstärkung der Kühlwirkung von 5-Methyl-2-(propan-2-yl)cyclohexyl-N-ethyloxamat auf der Haut oder einer Schleimhaut, ein Verfahren zum Erzeugen einer verstärkten Kühlwirkung von 5-Methyl-2-(propan-2-yl)cyclohexyl-N-ethyloxamat auf der Haut oder einer Schleimhaut sowie ein Verfahren zum Erzeugen einer entsprechenden Kühlmischung.

### Hintergrund der Erfindung:

Kühlsubstanzen werden im Personal Care Bereich für die Applikation auf Haut, Haar und Schleimhäuten verwendet. Zu diesen Applikationen zählen Lotionen und Cremes, Hautreiniger, Shampoos, Haarspülungen, Reinigungstücher, Damenbinden, Tampons, Windeln sowie andere kosmetische Produkte wie Lippenstifte oder Eau de Toilette. Eine Vielzahl von Kühlsubstanzen natürlicher sowie synthetischer Herkunft wurde beschrieben. Die bekannteste Substanz unter diesen ist Menthol, insbesondere I-Menthol, welches zunächst in Pfefferminzöl gefunden wurde. Menthol bindet an den Rezeptor TRPM8 (*Transient Receptor Potential Melastatin 8*) der auch unter dem Namen CMR (*Cold Menthol Receptor 1*) bekannt ist. Dieser Rezeptor gehört zu der Familie der TRP's (*Transient Receptor Potential Ion Channels*) und wird in spezifischen, peripheren Neuronen exprimiert. Er bildet dort eine Pore bestehend aus vier Proteinmonomeren. Kalte Temperaturen sowie die Bindung von Kühlsubstanzen an den Kanal öffnen diesen und erlauben somit den Einstrom von Calcium- und Natriumionen durch die Membran. Dieser Ionenfluß resultiert in einer Depolarisation der Membran und der Generierung eines Aktionspotentials welches im Gehirn in eine Kälteempfindung umgesetzt wird (Clapham DE et al 2005, Pharmacological reviews, 57 (4), 427-450; Peier AM et al 2005, Cell, 108 (5), 705-15). Verschiedene Mentholderivate wurden beschrieben welche die Öffnung des TRPM8 induzieren (British Patent 1971 # 1315761 Watson H.R., J. Soc. Cosmet. Chem. 29, 1978, 185-200; Furrer S.M., Chem. Percept. 1, 2008, 119-126), dennoch gibt es auch Strukturen die Kühlwirkung besitzen und nicht auf Menthol basieren wie Icilin (Wei E.T., J. Pharm. Pharmacol. 35, 1983, 110-112; WO 2004/026840), WS-23 oder Substanzen die in WO 2007/019719 genannt sind.

Aktivatoren des TRPM8 können abstoßende Wirkung auf Insekten haben (WO 2002/015692, WO 2004/000023, US 2004/0028714) und auch beruhigende Eigenschaften bei der Behandlung von entzündungsbasiertem Schmerz und Hyperalgesie sowie eine überaktiven Blase aufweisen (Beck B. Cell Calcium, 41, 2007, 285-294; Levine J.D. Biochim. Biophys. Acta, Mol. Basis Dis. 1772, 2007, 989-1003; Mukerji G., BMC Urology 6, 2006, 6; US 2003/0207904; US 2005/6893626, Lashinger ES. Am. J. Physiol. Renal Physiol. 295, 2008, 303-810).

Verschiedene Untersuchungen konnten des Weiteren zeigen, dass einige Aktivatoren vom TRPM8 wachstumsinhibierende Wirkung auf Tumoren haben (Slominski A., Am. J. Physiol. 295, 2008, 293-295; Yamamura H, Am. J. Physiol. Cell Physiol. 295, 2008, 296-301).

Viele Kühlsubstanzen haben bedingt durch ihre strukturelle Nähe zu Menthol einen mintähnlichen Geruch und sind daher für topische Anwendungen ungeeignet. Zudem muss bei vielen physiologischen Kühlsubstanzen eine hohe Konzentration von mehr als 3% eingesetzt werden, um einen spürbaren Kühleffekt zu induzieren. Hohe Konzentrationen von Kühlsubstanzen können auf der Haut ungewollte Nebeneffekte wie Stechen und Brennen verursachen (Wasner G., Brain, 2004, 127, 1159-1171; Green BG, Behav Brain Res, 2007, 176, 284-291).

Aufgabe der vorliegenden Erfindung war es vor diesem Hintergrund eine Wirkstoffkombination anzugeben, die es ermöglicht, einen geeigneten Kühlwirkstoff für topische Anwendungen in verringerter Konzentration einzusetzen.

Diese Aufgabe wird gelöst durch eine Kühlmischung, umfassend oder bestehend aus:
(a) 5-Methyl-2-(propan-2-yl)cyclohexyl-N-ethyloxamat (Formel I) 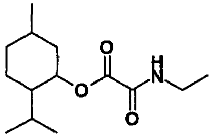 und
(b) ein, zwei, drei oder mehreren Polyolen, ausgewählt aus der Gruppe A bestehend aus verzweigten oder unverzweigten Alkandiolen und verzweigten oder unverzweigten Alkantriolen mit jeweils 3-12 Kohlenstoffatomen.

5-Methyl-2-(propan-2-yl)cyclohexyl-N-ethyloxamat ist als Kühlwirkstoff aus der EP 2 033 688 A2 bekannt. Die Erfinder konnten nun überraschend zeigen, dass die Kombination dieser Verbindung mit verzweigten oder unverzweigten Alkandiolen oder verzweigten oder unverzweigten Alkantriolen mit jeweils 3 bis 12 Kohlenstoffatomen zu einer Verstärkung der Kühlwirkung bei topischer Anwendung führt. Hierbei handelt es um keinen additiven Effekt, da die entsprechenden Diole und Triole keine Kühlwirkung besitzen.

Eine Kühlmischung im Sinne dieser Erfindung umfasst dabei eine ausreichende Menge an 5-Methyl-2-(propan-2-yl)cyclohexyl-N-ethyloxamat, so dass eine Kühlwirkung bei topischer Anwendung wahrgenommen wird. Bevorzugt umfasst eine entsprechende Kühlmischung eine ausreichende Menge an 5-Methyl-2-(propan-2-yl)cyclohexyl-N-ethyloxamat, so dass eine Kühlwirkung bei topischer Anwendung auch dann wahrgenommen werden würde, wenn sämtliche anderen Kühlwirkstoffe eine Konzentration von 0 besitzen. Selbstverständlich ist es im Sinne der Erfindung möglich, dass die Kühlmischung nur eine so geringe Menge an 5-Methyl-2-(propan-2-yl)cyclohexyl-N-ethyloxamat umfasst, dass diese nur durch Verstärkung durch die entsprechenden Polyole wahrnehmbar ist.

Im Zweifelsfall ist eine Wahrnehmbarkeit der Kühlwirkung durch einen Paneltest zu bestimmen, bei dem die entsprechende Mischung auf die Haut von wenigstens 10 Probanden aufgetragen wird. Sofern dabei, bevorzugt in Kombination mit einem Blindtest, von 90 % der Panellisten eine Kühlwirkung bestätigt wird, entspricht das bevorzugt den Kriterien für das Vorhandensein einer entsprechenden Wirkung. Topische Anwendungen im Sinne dieses Textes sind Anwendungen, bei denen der entsprechende Wirkstoff bzw. die Wirkstoffkombination mit der Haut oder einer Schleimhaut in Kontakt gebracht wird.

Bevorzugt ist eine erfindungsgemäße Kühlmischung, wobei wenigstens ein Teil des 5-Methyl-2-(propan-2-yl)-cyclohexyl-N-ethyloxamat in der (1*R*,2*S*,5*R*)-Konfiguration vorliegt (Formel II):

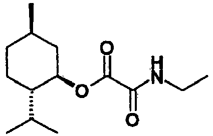

Das 1*R*,2*S*,5*R-isomer des* 5-Methyl-2-(propan-2-yl)cyclohexyl-N-ethyloxamat ist für eine Verstärkung der Kühlwirkung besonders gut zugänglich und ist außerdem über L-Menthol als Ausgangsverbindung in wirtschaftlicher Weise zu synthetisieren.

Dementsprechend ist eine erfindungsgemäße Kühlmischung bevorzugt, wobei der Anteil 5-Methyl-2-(propan-2-yl)cyclohexyl-N-ethyloxamat, das in der (1 R,2S,5R)-Konfiguration vorliegt, bezogen auf den Gesamtanteil an 5-Methyl-2-(propan-2-yl)cyclohexyl-N-ethyloxamat ≥ 45%, bevorzugt ≥ 70% und besonders bevorzugt ≥ 97% beträgt.

Erfindungsgemäß bevorzugt ist alternativ oder zusätzlich zu den bevorzugten Formen eine erfindungsgemäße Kühlmischung, wobei das Verhältnis der Stoffmenge von 5-Methyl-2-(propan-2-yl)cyclohexyl-N-ethyloxamat zu der Stoffmenge des Polyols oder der Polyole der Gruppe A gemeinsam 1:20 bis 1:0,1, bevorzugt 1:10 bis 1:1:0,5 und besonders bevorzugt 1:5 bis 1:1 beträgt.

Im Sinne der Erfindung ist weiter bevorzugt, dass in der erfindungsgemäßen Kühlmischung ein oder das Polyol oder mehrere oder alle Polyole der Gruppe A ausgewählt ist oder sind aus der Gruppe bestehend aus den verzweigten oder unverzweigen Alkandiolen mit 3 bis 12 Kohlenstofatomen.

Ferner ist für die erfindungsgemäße Kühlmischung bevorzugt, dass bei einem oder dem Alkanpolyol der Gruppe A oder mehreren oder allen Alkanpolyolen der Gruppe A beide oder wenigstens zwei der Hydroxygruppen zueinander vicinal stehen.

Eine erfindungsgemäße Kühlmischung ist darüber hinaus bevorzugt, für die als Bestandteil (b) ausschließlich ein oder mehrere verzweigte oder unverzweigte 1,2-Alkandiole mit 5-12 Kohlenstoffatomen eingesetzt werden.

Die zuletzt genannten bevorzugten Varianten der Kühlmischung führen allein oder in Kombination der bevorzugten Merkmale jeweils zu einer verbesserten Verstärkungswirkung für den eigentlichen Kühlwirkstoff 5-Methyl-2-(propan-2-yl)cyclohexyl-N-ethyloxamat. Bevorzugte Diole oder Triole für die erfindungsgemäße Kühlmischung als Bestandteil (b) Teil des Bestandteiles (b) sind 1,2-Pentandiol, 1,2-Hexandiol, 1,2-Heptandiol, 1,2-Oktandiol, 1,2-Nonandiol, 1,2-Dekandiol, 1,2-Dodekandiol und Glycerol.

Besonders bevorzugt ist in diesem Zusammenhang, dass in der Kühlmischung der Bestandteil (b) n-1,2-Pentandiol umfasst oder daraus besteht.

Sofern die erfindungsgemäße Kühlmischung n-1,2-Pentandiol umfasst ist es bevorzugt, dass das Verhältnis der Stoffmenge von 5-Methyl-2-(propan-2-yl)cyclohexy-N-ethyloxamat zu der Stoffmenge von n-1,2-Pentandiol 1:20 bis 1:0,1, bevorzugt 1:10 bis 1:0,5 und besonders bevorzugt 1:5 bis 1:1 beträgt.

In diesem Stofmengenverhältnis entfaltet n-1,2-Pentandiol (auch als Pentylenglycol) seine verstärkende Wirkung besonders gut. Außerdem sind entsprechende Verhältnisse hinsichtlich ihres Kosten-Nutzen-Faktors als besonders wirtschaftlich anzusehen.

Weiter bevorzugt ist eine erfindungsgemäße Kühlmischung, die als weiteren Bestandteil eine Verbindung umfasst, die auf Wärmerezeptoren der Haut oder Schleimhäute antagonistisch wirkt, mithin die Wärmeempfindung herabsetzt. Bevorzugt wird als solche Verbindung *trans-* 4-Tert Butylcyclohexanol eingesetzt

Bevorzugt werden diese Verbindungen, insbesondere *trans-* 4-Tert Butylcyclohexanol in einem Stoffmengenverhältnis zu der Summe der Diole und Triole von 100:1 bis 1:1000 , weiter bevorzugt von 10:1 bis 1:100 und besonders bevorzugt von 1:1 bis 1:10.

Durch eine Beifügung entsprechender Wärmerezeptorantagonisten ist es möglich, die empfundene Kühlwirkung, die über Kühlrezeptoren vermittelt wird, subjektiv noch einmal zu steigern, in dem die Empfindlichkeit der Wärmerezeptoren verringert wird

Teil der Erfindung ist auch eine kosmetische Zubereitung umfassend eine erfindungsgemäße Kühlmischung.

Ebenfalls Teil der Erfindung ist ein Hygieneartikel umfassend eine erfindungsgemäße Kühlmischung.

Bevorzugte kosmetische Zubereitungen sind Haarpflegemittel und Hautpflegemittel, bevorzugte Hygieneartikel sind Damenbinden, Tampons und Windeln, insbesondere Babywindeln.

Für die kosmetischen Zubereitungen ist es bevorzugt, dass der Anteil an 5-Methyl-2-(propan-2-yl)cyclohexy-N-ethyloxamat an der Gesamtzubereitung im Bereich von 0,001 bis 20, bevorzugt von 0,01 bis 10 und besonders von 0,1 Bis 5 Gew.-% beträgt jeweils bezogen auf das Gesamtgewicht der kosmetischen Zubereitung.

Für Hygieneartikel ist es bevorzugt, dass der Anteil 5-Methyl-2-(propan-2-yl)cyclohexy-N-ethyloxamat an der Gesamtzubereitung im Bereich von 0,000001 bis 20, bevorzugt von 0,0001 bis 10 und besonders von 0,001 bis 5 Gew.-% beträgt jeweils bezogen auf das Gesamtgewicht des Hygieneartikels.

### Kühlende Haut- und Haarpflegemittel

Nach einer bevorzugten Ausführungsform handelt es sich bei den erfindungsgemäßen Zubereitungen um ein kühlendes Haut- oder Haarpflege- oder -reinigungsmittel.

Bevorzugte Haut- oder Haarreinigungszubereitungen sind Seifen von flüssiger bis gelförmiger Konsistenz, insbesondere Transparentseifen, Luxusseifen, Deoseifen, Cremeseifen, Babyseifen, Hautschutzseifen, abrasive Seifen und Syndets, pastöse Seifen, Schmierseifen, Waschpasten, Peelingseifen, flüssige Wasch-, Dusch- und Badepräparate, wie Waschlotionen, Duschbäder und Duschgele, Schaumbäder, Ölbäder und Scrub-Präparate, Rasierschäume, -lotionen und -cremes.

Nach einer weiteren bevorzugten Ausführungsform handelt es sich dabei um ein Duschgel, eine Shampoo-Formulierung oder ein Badepräparat. Solche erfindungsgemäßen Zubereitungen enthalten wenigstens eine erfindungsgemäße Kühlmischung sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

### i) Spezielle Ausgestaltungen für erfindungsgemäße Zubereitungen zur Auftragung auf die Haut (Hautpflegemittel):

Geeignete erfindungsgemäße hautkosmetische Zubereitungen sind z. B. Gesichtswässer, Gesichtsmasken, Deodorantien und andere kosmetische Lotionen, Zubereitungen für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die erfindungsgemäßen Zubereitungen verwendet werden in Nose-Strips zur Porenreinigung, in Antiaknemitteln, Repellents, Rasiermitteln, After- und Pre-Shave-Pflegemitteln, After-Sun-Pflegemitteln, Haarentfernungsmitteln, Handspülmitteln, Haarfärbemitteln, Intimpflegemitteln, Fußpflegemitteln sowie in der Babypflege.

Bei den erfindungsgemäßen Hautpflegemitteln handelt es sich bevorzugt um W/O- oder O/W-Hautcremes, Tag- und Nachtcremes, Augencremes, Gesichtscremes, Antifaltencremes, Sonnenschutzcremes, Feuchthaltecremes, Bleichcremes, Selbstbräunungscremes, Vitamincremes, Hautlotionen, Pflegelotionen und Feuchthaltelotionen.

Je nach Anwendungsgebiet können die erfindungsgemäßen Zubereitungen in einer zur Hautpflege geeigneten Form, wie z. B. als Creme, Schaum, Gel, Stift, Mousse, Milch, Spray (Pumpspray oder treibmittelhaltiges Spray) oder Lotion appliziert werden.

Die erfindungsgemäßen Zubereitungen können neben den erfindungsgemäßen Kühlmischungen und geeigneten Trägern noch weitere in der Hautkosmetik übliche Wirkstoffe und Hilfsstoffe, insbesondere wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Enzyme, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive.

Bevorzugte Öl- und Fettkomponenten der erfindungsgemäßen hautkosmetischen Zubereitungen sind die zuvor genannten mineralischen und synthetischen Öle, wie z. B. Paraffine, Silikonöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie z. B. Pigmenten, können die erfindungsgemäßen hautkosmetischen Zubereitungen zusätzlich auch konditionierende Substanzen auf Basis von Silikonverbindungen enthalten. Geeignete Silikonverbindungen sind insbesondere Polyalkylsiloxane, Polyarylsiloxane, Polyarylalkylsiloxane, Polyethersiloxane oder Silikonharze.

Die Herstellung der erfindungsgemäßen kosmetischen Zubereitungen erfolgt nach üblichen, dem Fachmann bekannten Verfahren.

Zur Herstellung der erfindungsgemäßen Zubereitungen können die Wirkstoffe mit einem geeigneten Hilfsstoff (Exzipient) vermischt oder verdünnt werden. Exzipienten können feste, halbfeste oder flüssige Materialien sein, die als Vehikel, Träger oder Medium für die Wirkstoffe dienen können. Die Zumischung weiterer Hilfsstoffe erfolgt gewünschtenfalls in der dem Fachmann bekannten Weise. Weiterhin sind die Polymere und Dispersionen geeignet, als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Bevorzugt liegen die erfindungsgemäßen kosmetischen Zubereitungen in Form von Emulsionen insbesondere als Wasser-in-ÖI (W/O)- oder Öl-in-Wasser (O/W)-Emulsionen vor. Es ist aber auch möglich, andere Formulierungsarten zu wählen, beispielsweise, Gele, Öle, Oleogele, multiple Emulsionen, beispielsweise in Form von W/O/W- oder O/W/O-Emulsionen, wasserfreie Salben bzw. Salbengrundlagen usw. Auch emulgatorfreie Formulierungen wie Hydrodispersionen, Hydrogele oder eine Pickering-Emulsion sind vorteilhafte Ausführungsformen.

Die Herstellung von Emulsionen erfolgt nach bekannten Methoden. Die Emulsionen enthalten neben wenigstens einem erfindungsgemäßen Wirkstoff in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion als W/O-Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein Polyelektrolytkomplex eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Rizinusöl, Sesamöl, Olivenöl, Jojobaöl, Karite-Öl, Hoplostethus-Öl, mineralische Öle, deren Destillationsbeginn unter Atmosphärendruck bei ca. 250°C und deren Destillationsendpunkt bei 410°C liegt, wie z. B. Vaselinöl, Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Silikonöle, wie Dimethylpolysiloxan, Methylphenylpolysiloxan und das Silikonglykol-Copolymer, Fettsäuren und Fettalkohole enthalten.

Neben den erfindungsgemäßen Kühlmischungen können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Zubereitung als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride in Betracht.

Nach einer weiteren bevorzugten Ausführungsform handelt es sich bei den erfindungsgemäßen Zubereitungen um ein Duschgel, eine Shampooformulierung oder ein Badepräparat.

Solche Formulierungen enthalten wenigstens eine erfindungsgemäße Kühlmischung sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidanzien sowie Verdickern/Gelbildnern, Hautkonditioniermitteln und Feuchthaltemitteln.

In den erfindungsgemäßen Wasch-, Dusch- und Badezubereitungen können alle in Körperreinigungsmitteln üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten, im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt ca. 6 bis 60 Mole auf ein Mol Alkohol. Ferner sind Alkylaminoxide, Mono-oder Dialkylalkanolamide, Fettsäureester von Polyethylenglykolen, ethoxylierte Fettsäureamide, Alkylpolyglycoside oder Sorbitanetherester geeignet.

Außerdem können die erfindungsgemäßen Wasch-, Dusch- und Badezubereitungen übliche kationische Tenside enthalten, wie z. B. quaternäre Ammoniumverbindungen, beispielsweise Cetyltrimethylammoniumchlorid.

Weiterhin können die Duschgel-/Shampooformulierungen Verdicker, wie z. B. Kochsalz, PEG-55, Propylenglykol-Oleat, PEG-120-Methylglucosedioleat und andere sowie Konservierungsmittel, weitere Wirk- und Hilfsstoffe und Wasser enthalten.

Erfindungsgemäße kosmetische Zubereitungen im Sinne eines Hautpflegemittels können auch Sonnenschutzzubereitungen sein. Dem Fachmann ist klar, dass Sonnenschutzzubereitungen auch zu anderen Zwecken als zur Hautpflege eingesetzt werden. Im Sinne der vorliegenden Anmeldung werden Sonnenschutzzubereitungen jedoch als Hautpflegemittel (im weitesten Sinne) aufgefasst. Erfindungsgemäße Sonnenschutzzubereitungen umfassen eine erfindungsgemäße Kühlmischung.

Vorteilhaft enthalten diese Zubereitungen mindestens einen UVA-Filter und/oder mindestens einen UVB-Filter und/oder mindestens ein anorganisches Pigment. Die Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol bilden.

Die erfindungsgemäßen Sonnenschutzzubereitungen können besonders vorteilhaft mit Substanzen kombiniert werden, die UV-Strahlung absorbieren oder reflektieren, wobei die Gesamtmenge der Filtersubstanzen von 0,01 Gew.-% bis 40 Gew.-%, vorzugsweise 0,1% bis 10 Gew.-%, insbesondere 1,0 bis 5,0 Gew.-% beträgt, bezogen auf das Gesamtgewicht der Zubereitungen, um kosmetische Zubereitungen zur Verfügung zu stellen, die das Haar bzw. die Haut vor ultravioletter Strahlung schützen. Vorteilhaft enthalten diese Zubereitungen mindestens einen UVA-Filter und/oder mindestens einen UVB-Filter und/oder mindestens ein anorganisches Pigment, so dass ein Lichtschutzfaktor von zumindest ≥ 2 (bevorzugt ≥ 5) erreicht wird. Diese erfindungsgemäßen Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

### Vorteilhafte UV-Filter sind

UVB-Filter wie z.B.:
- p-Aminobenzoesäure
- p-Aminobenzoesäureethylester (25 Mol) ethoxyliert
- p-Dimethylaminobenzoesäure-2-ethylhexylester
- p-Aminobenzoesäureethylester (2 Mol) N-propoxyliert
- p-Aminobenzoesäureglycerinester
- Salicylsäure-homomenthylester (Homosalate) (Neo Heliopan®HMS)
- Salicylsäure-2-ethylhexylester (Neo Heliopan^{®}OS)
- Triethanolaminsalicylat
- 4-Isopropylbenzylsalicylat
- Anthranilsäurementhylester (Neo Heliopan^{®}MA)
- Diisopropylzimtsäureethylester
- p-Methoxyzimtsäure-2-ethylhexylester (Neo Heliopan^{®}AV)
- Diisopropylzimtsäuremethylester
- p-Methoxyzimtsäureisoamylester (Neo Heliopan^{®}E 1000)
- p-Methoxyzimtsäure-diethanolaminsalz
- p-Methoxyzimtsäure-isopropylester
- 2-Phenylbenzimidazolsulfonsäure und Salze (Neo Heliopan^{®}Hydro)
- 3-(4'-Trimethylammonium)-benzyliden-bornan-2-on-methylsulfat
- ß-Imidazol-4(5)-acrylsäure (Urocaninsäure)
- 3-(4'-Sulfo)benzyliden-bornan-2-on und Salze
- 3-(4'-Methylbenzyliden)-d,l-campher (Neo Heliopan^{®}MBC)
- 3-Benzyliden-d,l-campher
- N-[(2 und 4)-[2-(oxoborn-3-yliden)methyl]benzyl]-acrylamid-Polymer
- 4,4'-[(6-[4-(1,1-Dimethyl)-aminocarbonyl)-phenylamino]-1,3,5-triazin-2,4-diyl)diimino]-bis-(benzoesäure-2-ethylhexylester) (Uvasorb^{®}HEB)
- Benzylidenmalonat-Polysiloxan (Parsol^{®}SLX)
- Glyceryl-ethylhexanoat-dimethoxycinnamat
- Dipropylenglykolsalicylat
- Tris(2-etylhexyl)-4,4',4"-(1,35-triazin-2,4,6-triyltriimino)tribenzoat (Uvinul^{®}T150)

Breitband-Filter wie z.B.
- 2-Ethylhexyl-2-cyano-3,3-diphenylacrylat (Neo Heliopan^{®}303)
- Ethyl-2-cyano-3,3'-diphenylacrylat
- 2-Hydroxy-4-methoxybenzophenon (Neo Heliopan^{®}BB)
- 2-Hydroxy-4-methoxybenzophenon-5-sulfonsäure
- Dihydroxy-4-methoxybenzophenon
- 2,4-Dihydroxybenzophenon
- Tetrahydroxybenzophenon
- 2,2'-Dihydroxy-4,4'-dimethoxybenzophenon
- 2-Hydroxy-4-n-octoxybenzophenon
- 2-Hydroxy-4-methoxy-4'-methylbenzophenon
- Natrium-hydroxymethoxybenzophenon-sulfonat
- Dinatrium-2,2'-dihydroxy-4,4'-dimethoxy-5,5'-disulfo-benzophenon
- Phenol, -(2H-benzotriazol-2-yl)-4-methyl-6-(2-methyl-3(1,3,3,3-tetramethyl-1-(trimethylsilyl)-oxy)-disiloxyanyl)-propyl), (Mexoryl^{®}XL)
- 2,2'-Methylen-bis-(6-(2H-benztriazol-2-yl)-4-1,1,3,3-tetramethylbutyl)-phenol), (Tinosorb^{®}M)
- 2,4-bis-[4-(2-ethylhexyloxy)-2-hydroxyphenyl]-1,3,5-triazin
- 2,4-Bis-[{(4-(2-Ethyl-hexyloxy)-2-hydroxy}-phenyl]-6-(4-methoxyphenyl)-1,3,5-triazin, (Tinosorb^{®}S)
- 2,4-Bis-[{(4-(3-sulfonato)-2-hydroxy-propyloxy)-2-hydroxy}-phenyl]-6-(4-methoxyphenyl)-1,3,5-triazin-Natriumsalz
- 2,4-Bis-[{(3-(2-Propyloxy)-2-hydroxy-propyloxy)-2-hydroxy}-phenyl]-6-(4-methoxy-phenyl)-1,3,5-triazin
- 2,4-Bis-[{4-(2-Ethyl-hexyloxy)-2-hydroxy}-phenyl]-6-[4-(2-methoxyethyl- carbonyl)-phenylamino]-1,3,5-triazin
- 2,4-Bis-[{4-(3-(2-propyloxy)-2-hydroxy-propyloxy)-2-hydroxy}-phenyl]-6-[4-(2-ethylcarboxyl)-phenylamino]-1,3,5-triazin
- 2,4-Bis-[{4-(2-Ethyl-hexyloxy)-2-hydroxy}-phenyl]-6-(1-methyl-pyrrol-2-yl-)-1,3,5-triazin
- 2,4-Bis-[{4-tris-(trimethylsiloxy-silylpropyloxy)-2-hydroxy}-phenyl]-6-(4-methoxyphenyl)-1,3,5-triazin
- 2,4-Bis-[{4-(2"-Methylpropenyloxy)-2-hydroxy}-phenyl]-6-(4-methoxyphenyl)-1,3,5-triazin
- 2,4-Bis-[{4-(1',1',1',3'5',5',5'-Heptamethylsiloxy-2"-methyl-propyloxy)-2-hydroxy}-phenyl]-6-(4-methoxyphenyl)-1,3,5-triazin

UVA-Filter wie z.B.
- 4-Isopropyldibenzoylmethan
- Terephthalyliden-dibornansulfonsäure und Salze (Mexoryl^{®}SX)
- 4-t-Butyl-4'-methoxy-dibenzoylmethan (Avobenzon) / (Neo Heliopan^{®}357)
- Phenylen-bis-benzimidazyl-tetrasulfonsäure-dinatriumsalz (Neo Hellopan^{®}AP)
- 2,2'-(1,4-Phenylen)-bis-(1H-benzimidazo)-4,6-disulfonsäure), Mononatriumsalz
- 2-(4-Diethylamino-2-hydroxybenzoyl)-benzoesäure-hexylester (Uvinul^{®} A Plus)
- Indanylidenverbindungen gemäß DE 100 55 940 (= WO 02/38537)

Besonders zur Kombination geeignete UV-Absorber sind dabei
- p-Aminobenzoesäure
- 3-(4'-Trimethylammonium)-benzyliden-bornan-2-on-methylsulfat
- Salicylsäure-homomenthylester (Neo Heliopan^{®}HMS)
- 2-Hydroxy-4-methoxy-benzophenon (Neo Heliopan^{®}BB)
- 2-Phenylbenzimidazolsulfonsäure (Neo Heliopan^{®}Hydro)
- Terephthalyliden-dibornansulfonsäure und Salze (Mexory^{®}SX)
- 4-tert.-Butyl-4'-methoxydibenzoylmethan (Neo Heliopan^{®}357)
- 3-(4'-Sulfo)benzyliden-bornan-2-on und Salze
- 2-Ethylhexyl-2-cyano-3,3-diphenylacrylat (Neo Heliopan^{®}303)
- N-[(2 und 4)-[2-(oxoborn-3-yliden)methyl]benzyl]-acrylamid-Polymer
- p-Methoxyzimtsäure-2-ethylhexylester (Neo Heliopan^{®}AV)
- p-Aminobenzoesäure-ethylester (25 Mol) ethoxyliert
- p-Methoxyzimtsäure-isoamylester (Neo Heliopan^{®}E1000)
- 2,4,6-Trianilino-(p-carbo-2'-ethylhexyl-1'-oxy)-1,3,5-triazin (Uvinul^{®}T150)
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl^{®}XL)
- 4,4'-[(6-[4-(1,1-Dimethyl)-aminocarbonyl)-phenylamino]-1,3,5-triazin-2,4-diyl)-diimino]-bis-(benzoesäure-2-ethylhexylester), (UvasorbHEB)
- 3-(4'-Methylbenzyliden)-d,l-campher (Neo Helipan^{®}MBC)
- 3-Benzylidencampher
- Salicylsäure-2-ethylhexylester (Neo Helipan^{®}OS)
- 4-Dimethylaminobenzoesäure-2-ethylhexylester (Padimate O)
- Hydroxy-4-methoxy-benzophenon-5-sulfonsäure und Na-Salz
- 2,2'-Methylen-bis-(6-(2H-benztriazol-2-yl)-4-1,1,3,3-tetramethylbutyl)-phenol), (Tinosorb^{®}M)
- Phenylen-bis-benzimidazyl-tetrasulfonsäure-dinatriumsalz (Neo Heliopan^{®}AP)
- 2,4-Bis-[{(4-(2-Ethyl-hexyloxy)-2-hydroxy}-phenyl]-6-(4-methoxyphenyl)-1,3,5-triazin, (Tinosorb^{®}S)
- Benzylidenmalonat-Polysiloxan (Parsol^{®}SLX)
- Menthylanthranilat (Neo Heliopan^{®}MA)
- 2-(4-Diethylamino-2-hydroxybenzoyl)-benzoesäure-hexylester (Uvinul^{®} A Plus)
- Indanylidenverbindungen gemäß DE 100 55 940 (= WO 02/38537)

Vorteilhafte anorganischen Lichtschutzpigmenten sind feindisperse Metalloxide und Metallsalze, beispielsweise Titandioxide, Zinkoxid (ZnO), Eisenoxide (z.B. Fe₂O₃), Aluminiumoxid (Al₂O₃); Ceroxide (z. B. Ce₂O₃), Manganoxide (z. B. MnO), Zirkoniumoxid (ZrO₂), Siliciumoxid (SiO₂), Mischoxide der entsprechenden Metalle sowie Abmischungen aus solchen Oxiden, Bariumsulfat und Zinkstearat. Besonders bevorzugt handelt es sich um Pigmente auf der Basis von TiO₂ oder Zinkoxid. Die Partikel besitzen in bevorzugten Ausführungsformen einen mittleren Durchmesser von weniger als 100 nm, vorzugsweise ziwschen 5 und 50 nm und insbesondere bevorzugt ziwschen 15 und 30 nm. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d. h. hydrophilisiert oder hydrophobisiert vorliegen. Typische Beispiele sind gecoatete Titandioxide, wie z. B. Titandioxid T 805 (Degussa) oder Eusolex^{®} T2000 (Merck) oder gecoatetes Zinkoxid, wie z. B. Zinc Oxid NDM. Als hydrophobe Coatingmittel kommen dabei vor allem Silicone und dabei speziell Trialkoxyoctysilane oder Simethicone in Frage. In Sonnenschutzmitteln werden bevorzugt sogenannte Mikro- oder Nanopigmente eingesetzt. Vorzugsweise werden Zink-Mikro- oder -Nanopigmente eingesetzt.

Die Gesamtmenge an anorganischen Pigmenten, insbesondere hydrophoben anorganischen Mikropigmenten in den fertigen erfindungsgemäßen Zubereitungen liegt vorteilhaft im Bereich von 0,1 bis 30 Gew.-%, bevorzugt 0,1 bis 10,0, insbesondere 0.5 bis 6.0 Gew.-%, bezogen auf das Gesamtgewicht der Zubereitung.

### ii) Spezielle Ausgestaltungen für erfindungsgemäße Zubereitungen zur Auftragung auf das Haar

Nach einer weiteren bevorzugten Ausführungsform handelt es sich bei den erfindungsgemäßen Zubereitungen um ein Haarpflegemittel (Haarbehandlungsmittel).

Vorzugsweise liegen die erfindungsgemäßen Haarpflegemittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-) Spray, (Aerosol-) Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden.

Die erfindungsgemäßen Haarpflegemittel können Alkohol enthalten, unter Alkohol sind alle in der Kosmetik üblichen Alkohole zu verstehen, z. B. Ethanol, Isopropanol, n-Propanol.

Weiterhin können sie alle in der Kosmetik bekannten Styling- und Conditioner-Polymere enthalten, die in Kombination mit den erfindungsgemäßen Kühlmischungen eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die erfindungsgemäßen Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon-Copolyole (CTFA) und aminofunktionelle Silikonverbindungen wie Amodimethicone (CTFA).

Die erfindungsgemäßen Haarpflegemittel können Treibmittel umfassen. Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4, Cetethe, z. B. Cetheth-1, Polyethylenglycolcetylether, Cetearethe, z. B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Der Einsatz von Gelbildnern kann von Vorteil sein, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid / Acrylamid-Copolymere, Steareth-10-Allylether, AcrylatCopolymere, Polyquatemium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44.

In den erfindungsgemäßen Haarpflegemitteln als Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxid-Einheiten, bevorzugt 1 bis 3 Ethylenoxid-Einheiten, im Molekül aufweisen.

Geeignet sind zum Beispiel Natriumlaurylsulfat, Ammoniumlaurysulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die erfindungsgemäßen Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Wirkstoffen eingesetzt werden.

Hierzu zählen beispielsweise die zuvor genannten kationischen Polymere mit der Bezeichnung Polyquaternium nach INCI, insbesondere Copolymere aus Vinylpyrrolidon/ N-Vinylimidazoliumsalzen (Luviquat FC, Luviquat HM, Luviquat MS, Luviquat Care), Copolymere aus N-Vinylpyrrolidon/Dimethylaminoethylmethacrylat, quaternisiert mit Diethylsulfat (Luviquat D PQ 11), Copolymere aus N-Vinylcaprolactam/N-Vinylpyrrolidon/N-Vinylimidazoliumsalzen (Luviquat D Hold), kationische Cellulosederivate (Polyquaternium-4 und -10), Acrylamidcopolymere (Polyquaternium-7). Ferner können Eiweißhydrolysate verwendet werden sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon-Kopolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

### Kühlende Pflaster als Hautpflegemittel

In der vorliegenden Erfindung werden Pflaster, die eine Kühlwirkung auf der Haut oder auf Schleimhäuten entfalten, auch als Hautpflegemittel verstanden. Selbstverständlich ist dem Fachmann klar, dass entsprechende Pflaster auch zu anderen Zwecken als zu Hautpflegezwecken eingesetzt werden können. Dies ist ausdrücklich in die Erfindung mit eingeschlossen.

Erfindungsgemäße Pflaster als Hautpflegmittel umfassen eine erfindungsgemäße Kühlmischung. Sie können in beliebiger Weise aufgebaut sein, beispielsweise nach dem Matrixsystem, dem Membransystem oder dem Vliessystem (Drug Dev. Ind. Pharm. 14 (1988), 183-209; Drug Dev. Ind. Pharm. 13 (1987), 589-651; Drugs of Today 23 (1987), 625-646).

Das Matrixsystem besteht in einfachster Weise aus 3 Teilen: der flexiblen Stützfolie, der den erfindungsgemäßen Kühlmischung enthaltenden Klebematrix und einer Abziehfolie. Falls eine nichtklebende Matrix verwendet wird, muss zur Haftung auf der Haut eine Randzone der Stützfolie mit Klebstoff versehen werden.

Ein Membransystem weist hingegen mindestens 5 Teile auf: eine flexible Stützfolie, ein Reservoir mit gelöster oder suspendierter erfindungsgemäßen Kühlmischung, eine Membran zur Steuerung der Wirkstofffreigabe, eine auf die Membran aufgezogene Klebeschicht und eine Abziehfolie.

Beim Vliessystem besteht die erfindungsgemäße Kühlmischung enthaltende Schicht aus einem saugfähigen Vlies oder porösen Polymer, das mit einer Wirkstofflösung oder -suspension getränkt ist. Diese Schicht, die fest mit der Stützfolie verbunden ist, wird durch eine Abziehfolie abgedeckt. Der Rand der Stützfolie ist, zur Applikation auf die Haut, mit Klebstoff versehen.

Grundsätzlich sind alle erfindungsgemäßen Kühlmischungen in dieser Weise formulierbar.

Die zu verwendenden Hilfsstoffe sind die für die Herstellung von Pflastern üblichen. Neben dem klebenden Agens, in der Regel ein Polymer mit einer Glastemperatur zwischen -70 und -10, insbesondere -55 und -25 °C, sowie einer Trägerfolie, die mit diesem klebenden Agens beschichtet wird, und dem Wirkstoff werden häufig Emulgatoren, Verdickungsmittel sowie Stoffe, die die Wirkstofffreigabe beeinflussen sollen, und andere Hilfsmittel zugesetzt.

Die klebrigen Polymeren mit den oben genannten niedrigen Glastemperaturen sind bekannt, beispielsweise aus den US-Patenten 2 973 282 und 3 307 544. Die selbstklebenden Bänder und Folien sollen auf bloßen Kontakt auf der menschlichen Haut kleben, doch soll die Kohäsion der Klebschicht und deren Adhäsion an der Trägerfolie größer sein als die Adhäsion an der Haut, so dass sie sich weitgehend rückstandslos wieder abziehen lassen. Es handelt sich in der Regel um Copolymerisate auf der Basis von Acryl- und Methacrylsäureestern von Alkoholen mit 2 bis 12, insbesondere 4 bis 8 Kohlenstoffatomen, die zahlreiche andere Comonomere einpolymerisiert enthalten können, beispielsweise (Meth)acrylsäure, (Meth)acrylnitril, (Meth)acrylamid, N-tert.-Butyl-(meth-)acrylamid, Vinylester wie Vinylacetat, -propionat oder -butyrat, andere Vinylverbindungen wie Styrol, ferner Butadien. Besonders hervorgehoben seien Butylacrylat und 2-Ethylhexylacrylat. Die Polymeren können durch Zusatz geringer Mengen Comonomerer mit 2 oder mehr copolymerisierbaren Doppelbindungen, also beispielsweise von Diacrylaten, wie Butandioldiacrylat, oder Divinylverbindungen, wie Divinylbenzol, oder durch Zusatz anderer Vernetzungsmittel, z.B. Melamin-Formaldehydharze, vernetzt sein. Als klebrige Polymere können ferner Polyisobutylene und Polyvinylether unterschiedlichen Molekulargewichts verwendet werden.

Die Teilchengröße der Dispersionen soll zwischen 50 und 500 nm, insbesondere zwischen 50 und 200 nm liegen. Die Teilchengröße und der Vernetzungsgrad können in bekannter Weise in Abhängigkeit von den Polymerisationsbedingungen und den Comonomeren eingestellt werden. Kleinere Teilchengrößen und ein erhöhter Vernetzungsgrad können eine Steigerung der Wirkstofffreisetzung bewirken.

Matrix-Pflaster können in üblicher Weise hergestellt werden, indem man den Wirkstoff in einer geeigneten Polymerlösung löst oder fein dispergiert und anschließend diese wirkstoffhaltige Selbstklebemasse mittels Walzen- oder Rakelauftragsverfahren zum Film auszieht. In einigen Fällen ist es zweckmäßig, den Wirkstoff vor der Zugabe zu der Polymerlösung in einem organischen Lösungsmittel, z.B. Ethanol oder Aceton, aufzulösen oder feinst zu dispergieren. Dadurch kann eine bessere Verteilung des Wirkstoffes im Polymer erzielt werden.

Die Pflaster können auch nach der deutschen Patentanmeldung P 38 07 283.1 hergestellt werden, indem man die Kühlmischung in feinpulvriger Form z. B. gebunden an einen Träger (Teilchengröße unter 200, insbesondere unter 50 µm) in die wässrige Latexdispersion einarbeitet, oder in einer wässrigen Emulgatorlösung dispergiert oder löst und diese Mischung der wässrigen Latexdispersion bei einer Temperatur von 10 bis 80, insbesondere 30 bis 70°C zumischt.

Zweckmäßig legt man die erfindungsgemäße Kühlmischung vor, gibt den Emulgator und Wasser zu und mischt dann mit der Polymerdispersion. Die so erhaltene Kühlmischung als Dispersion wird gegebenenfalls mit weiteren Hilfsstoffen versehen und, wie erwähnt, in an sich bekannter Weise auf einer Stützfolie zu einem Film ausgezogen und getrocknet. Die Trocknungstemperatur kann hierbei zwischen Raumtemperatur und 100°C liegen, wobei ein Optimum zwischen angestrebter schneller Trocknung und zu vermeidender Blasenbildung im Film sowie thermischer Belastung des Wirkstoffs im Allgemeinen bei 35 bis 45°C liegt.

Dieses Verfahren hat den großen Vorteil der Vermeidung von organischen Lösungsmitteln. Jedoch kommen im Prinzip auch alle anderen üblichen Herstellverfahren für Matrix-Pflaster in Betracht.

Die resultierenden Filme haben Dicken von 10 bis 800, bevorzugt 50 bis 300 µm. Die Filmherstellung kann kontinuierlich oder diskontinuierlich erfolgen. Das Auftrageverfahren kann mehrmals wiederholt werden, bis der Film die gewünschte Dicke erreicht hat. Die klebrige Polymerschicht enthält die Bestandteile a) und b) der erfindungsgemäßen Kühlmischung in einer aufsummierten Konzentration im Bereich von 1 bis 40, insbesondere 5 bis 25 Gew.-% bezogen auf die Gesamtmasse der klebrigen Polymerschicht. Die gleiche Konzentration gilt auch für die Reservoirflüssigkeit beim Membransystem (bezogen auf die Gesamtmasse der Reservoirflüssigkeit) und für die Kühlmischungslösung oder - Dispersion, mit der beim Vliessystem das Vlies oder das poröse Polymere getränkt wird (bezogen auf die Gesamtmasse der Lösung).

Als Emulgatoren sowohl für die Kühlmischung wie auch die Polymeren werden die hierfür üblichen Tenside verwendet, wie das Natriumsalz von längerkettigen Fettsäuren und des Schwefelsäurehalbesters eines (gegebenenfalls oxethylierten) Fettalkohols als Beispiele für anionische Tenside sowie polyoxethylierte Alkylphenole und längerkettige Fettalkohole (z.B. Hexadecan-(1)-ol) und Glycerin-Fettsäurepartialester als Beispiele für nichtionische Tenside und Coemulgatoren.

Die gewünschte Viskosität der ausziehfertigen Masse kann z.B. mit Polyacrylsäuren oder Cellulosederivaten eingestellt werden.

Als zusätzliche Vernetzungsmittel die die Kohäsion und damit die Klebeeigenschaften der Filme verbessern, können z.B. Melamin-Formaldehydharze verwendet werden.

Im Sinne einer Verbesserung der Wirkstofffreisetzung wirken Quellstoffe wie Polyvinylpyrrolidon, Cellulosederivate oder Polyacrylate, da der Film vermehrt Wasser aufnehmen kann und dadurch der Diffusionswiderstand sinkt. Die Freisetzung der Wirkstoffe kann ferner verbessert werden durch den Zusatz von hydrophilen Weichmachern wie Glycerin, 1,2-Propandiol der Polyethylenglykolen und lipophilen Weichmachern wie Triacetin, Dibutylphthalat oder Isopropylmyristat.

Matrixpflaster ergeben üblicherweise eine Wirkstofffreisetzung 1. Ordnung. Durch die Verwendung von Füllstoffen, die den Wirkstoff adsorbieren, wie Aerosil, mikrokristalline Cellulose oder Lactose, resultiert annähernd eine Freisetzung 0. Ordnung.

Die Stützfolie, auf die die kühlmischungshaltige Selbstklebemasse aufgetrocknet wird, ist zweckmäßig sowohl für den Wirkstoff wie für Wasserdampf praktisch undurchlässig. Sie kann z.B. aus einer Aluminium-Kunststoff-Verbundfolie, einer metallisierten Kunststofffolie, einer Kunststofffolie, die zur Wirkstoffseite hin mit einer Sperrschicht aus z.B. Polyvinylidenchlorid versehen ist, oder aus einer einfachen Kunststofffolie, z.B. Polyesterfolie, bestehen.

Die erfindungsgemäßen Pflaster, die nach dem Membransystem aufgebaut sind, werden ebenfalls in üblicher Weise hergestellt (z.B. EP 0 186 071 A2, US 4 262 003).

Die Herstellung der nach dem Vliessystem aufgebauten Pflaster erfolgt dadurch, dass auf der Stützfolie befestigte Vliese oder poröse Polymere mit einer Lösung oder Dispersion der Kühlmischung in einem hydrophilen oder lipophilen Lösungsmittel oder Lösungsmittelgemisch getränkt werden. Anschließend wird die undurchlässige Abziehfolie aufgebracht.

### Wirkstoffkombinationen

Gegebenenfalls können die erfindungsgemäßen Kühlmischungen mit weiteren bekannten Wirkstoffen, insbesondere auch solchen mit vergleichbarer Wirkung, kombiniert werden, das heißt sie können diese Wirkstoffe auch umfassen. Beispielsweise können diese mit bekannten kühlenden Verbindungen, wie z.B. Menthol, Menthon, N-Ethyl-p-menthancarboxamid (WS-3, auch Menthan-3-carbonsäure-N-ethylamid genannt), N-2,3-Trimethyl-2-isopropylbutanamid (WS-23), Menthyllactat (Frescolat® ML), Menthonglycerinacetal (Frescolat® MGA), Mono-menthylsuccinat (Physcool ®), Mono-menthylglutarat, O-Menthyl-glycerin, Menthyl-N,N-dimethylsuccinamat, N-(4-cyanomethylphenyl)-p-menthanecarboxamid, N-(2-(Pyridin-2-yl)ethyl)-3-p-menthanecarboxamid kombiniert werden.

Die erfindungsgemäßen Kühlmischungen, können bevorzugt mit den folgenden Kühlwirkstoffen kombiniert werden: Menthol und Mentholderivate (z.B. L-Menthol, D-Menthol, racemisches Menthol, Isomenthol, Neoisomenthol, Neomenthol) Menthylether (z.B. (I-Menthoxy)-1,2-propandiol, (I-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), N-(4-cyanomethylphenyl)-p-menthanecarboxamide, N-(2-(Pyridin-2-yl)ethyl)-3-p-menthanecarboxamide, Menthylcarbonate (z.B. Menthylpropylenglycolcarbonat, Menthylethylenglycolcarbonat, Menthylglycerincarbonat oder deren Mischungen), die Halbester von Mentholen mit einer Dicarbonsäure oder deren Derivate (z.B. Mono-Menthylsuccinat, Mono-Menthylglutarat, Mono-Menthylmalonat, O-Menthylbemsteinsäureester-N,N-(dimethyl)amid, O-Menthylbernsteinsäureesteramid), Menthancarbonsäureamide (z.B. Menthancarbonsäure-N-ethylamid [WS3], Nα-(Menthancarbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), N-(4-cyanomethylphenyl)-p-menthanecarboxamide, N-(2-(Pyridin-2-yl)ethyl)-3-p-menthanecarboxamide Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben).

Die erfindungsgemäßen Kühlmischungen, können besonders bevorzugt mit den folgenden Kühlwirkstoffen kombiniert werden: N-(4-cyanomethylphenyl)-p-menthanecarboxamide, N-(2-(Pyridin-2-yl)ethyl)-3-p-menthanecarboxamide, Menthylether (z.B. (I-Menthoxy)-1,2-propandiol, (I-Menthoxy)-2-methyl-1,2-propandion, polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbemsteinsäureester-N,N-(dimethyl)amid, O-Menthylbemsteinsäureesteramid), nicht erfindungsgemäße Menthancarbonsäureamide (z.B. Menthancarbonsäure-N-ethylamid [WS3], Nα-(Menthancarbonyl)glycinethylester [WS5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, die in WO 2004/026840 beschrieben sind).

### Antiirritantien

Erfindungsgemäße kosmetische Zubereitungen können auch entzündungshemmende und/oder rötungs- und/oder juckreizlindernde Wirkstoffe enthalten. Es können hierbei alle für kosmetische und/oder dermatologische Anwendungen geeigneten oder gebräuchlichen entzündungshemmenden bzw. rötungs- und/oder juckreizlindernden Wirkstoffe verwendet werden. Vorteilhaft werden als entzündungshemmende bzw. rötungs- und/oder juckreizlindernde Wirkstoffe steroidale entzündungshemmende Substanzen vom Kortikbsteroiden-Typ eingesetzt wie z.B. Hydrocortison, Hydrocortison-Derivate wie Hydrocortison-17-butyrat, Dexamethason, Dexamethasonphosphat, Methylprednisolon oder Cortison, wobei die Auflistung durch Zusatz weiterer steroidaler Entzündungshemmer erweiterbar ist. Auch nichtsteroidale Entzündungshemmer können eingesetzt werden. Beispielhaft erwähnt werden sollen hier Oxicame wie Piroxicam oder Tenoxicam; Salicylate wie Aspirin, Disalcid, Solprin oder Fendosal; Essigsäre-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Benoxaprofen oder Pyrazole wie Phenylbutazon, Oxyphenylbutazon, Febrazon oder Azapropazon. Alternativ können natürliche entzündungshemmende bzw. rötungs- und/oder juckreizlindernde Stoffe eingesetzt werden. Einsetzbar sind Pflanzenextrakte, spezielle hochwirksame Pflanzenextrakt-Fraktionen sowie aus Pflanzenextrakten isolierte hochreine Wirksubstanzen. Besonders bevorzugt sind Extrakte, Fraktionen und Wirksubstanzen aus Kamille, Aloe vera, Commiphora-Arten, Rubia-Arten, Weiden, Weidenröschen, Hafer, Calendula, Arnika, Johanniskraut, Geißblatt, Rosmarin, Melisse, Ingwer, Passiflora incarnata, Hamamelis, Pueraria, Dianthus oder Echinacea sowie Reinsubstanzen wie u.a. Bisabolol, Apigenin, Apigenin-7-glucosid, Rosmarinsäure, Boswelliasäure, Phytosterole, Glycyrrhizinsäure, Glabridin, Licochalkon A, Gingerole und Anthranilsäureamide wie insbesondere Avenanthramide oder Dianthramide. Die erfindungsgemäßen Zubereitungen können auch Gemische aus zwei oder mehreren antiinflammatorischen Wirkstoffen enthalten.

Die Menge der Antiirritantien (eine oder mehrere Verbindungen) in den Zubereitungen beträgt vorzugsweise 0,0001 bis 20 Gew.-%, besonders bevorzugt 0,0001 - 10 Gew.%, insbesondere 0,001 - 5 Gew.%, bezogen auf das Gesamtgewicht der Zubereitung.

### Antitranspirantien

Darüber hinaus können auch schweißhemmende Wirkstoffe (Antitranspirantien) besonders vorteilhaft mit den erfindungsgemäßen Zubereitungen eingesetzt werden. Als schweißhemmende Wirkstoffe kommen vor allem Aluminiumsalze wie Aluminiumchlorid, Aluminiumchlorhydrat, -nitrat, -sulfat, -acetat usw. zum Einsatz. Daneben kann aber auch die Verwendung von Zink-, Magnesium- und Zirkoniumverbindungen vorteilhaft sein. Für die Anwendung in kosmetischen und dermatologischen Antitranspirantien haben sich im wesentlichen die Aluminiumsalze und - in etwas geringerem Maße - Aluminium/Zirkoniumsalz-Kombinationen bewährt. Daneben erwähnenswert sind die teilneutralisierten und damit besser hautverträglichen, aber nicht ganz so wirksamen Aluminiumhydroxychloride. Neben Aluminiumsalzen kommen auch weitere Substanzen in Betracht wie zum Beispiel a) eiweißfällende Substanzen wie u.a. Formaldehyd, Glutaraldehyd, natürliche und synthetische Gerbstoffe sowie Trichloressigsäure, die einen oberflächlichen Verschluß der Schweißdrüsen herbeiführen b) Lokalanästhetika (u.a. verdünnte Lösungen von z.B. Lidokain, Prilokain oder Gemischen derartiger Substanzen), die durch Blockade der peripheren Nervenbahnen die sympathische Versorgung der Schweißdrüsen ausschalten, c) Zeolithe vom X-, A- oder Y-Typ, die neben der Reduktion der Schweißsekretion auch als Adsorbentien für schlechte Gerüche fungieren und d) Botulinustoxin (Toxin des Bakteriums Chlostridium botulinum) welches auch bei Hyperhidrose, einer krankhaft erhöhten Schweißsekretion, zum Einsatz gelangt und dessen Wirkung auf einer irreversiblen Blockierung der Freisetzung der für die Schweißsekretion relevanten Transmittersubstanz Acetylcholin beruht. Darüberhinaus können auch peptidische Botulinustoxin-Analoga in den erfindungsgemäßen Zubereitungen zum Einsatz gelangen.

Bevorzugte Hygieneartikel im Sinne der Erfindung sind Feuchtigkeitstücher, Damenbinden, Tampons und Erfrischungstücher, die eine erfindungsgemäße Kühlmischung umfassen. Selbstverständlich können die erfindungsgemäßen Kühlmischungen auch in einer der bevorzugten, oben beschriebenen Formen vorliegen. Ebenfalls können die erfindungsgemäßen Hygieneartikel erfindungsgemäße kosmetische Zubereitungen, insbesondere in den oben beschriebenen bevorzugten Varianten umfassen.

Bestandteil der Erfindung ist ferner die Verwendung eines, zweier, dreier oder mehrerer Polyole, ausgewählt aus der Gruppe A, bestehend aus verzweigten oder unverzweigten Alkandiolen und verzweigten oder unverzweigten Alkantriolen mit jeweils 3-12 Kohlenstoffatomen zur Verstärkung der Kühlwirkung von 5-Methyl-2-(propan-2-yl)cyclohexyl N-ethyloxamat (Formel I) 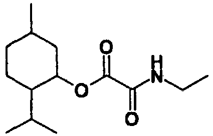 auf der Haut oder einer Schleimhaut.

Bei dieser Verwendung werden regelmäßig die erfindungsgemäßen Kühlmischungen entstehen oder eingesetzt. Dementsprechend ist es selbstverständlich, dass die Verwendung im Zusammenhang mit den bevorzugten, oben beschrieben erfindungsgemäßen Kühlmischungen, den erfindungsgemäßen kosmetischen Zubereitungen und den erfindungsgemäßen Hygieneartikeln erfolgen kann, insbesondere insbesondere in deren jeweiligen bevorzugten Ausführungsformen.

Teil der Erfindung ist ferner ein Verfahren zum Erzeugen einer verstärkten Kühlwirkung von 5-Methyl-2-(propan-2-yl)cyclohexyl-N-ethyloxamat (Formel I) 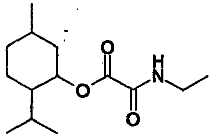 auf der Haut oder einer Schleimhaut, umfassend die folgenden Schritte:
a) Bereitstellen einer Kühlmischung oder einer kosmetischen Zubereitung und
b) Kontaktieren der Kühlmischung oder der Kosmetischen Zubereitung mit Haut oder einer Schleimhaut.

Bevorzugt ist in dem Zusammenhang, dass das Verfahren auf menschlicher Haut oder Schleimhaut angewendet wird. Es ist aber durchaus möglich, es auch für Tiere einzusetzen.

Bestandteil der Erfindung ist ferner ein Verfahren zum Erzeugen einer erfindungsgemäßen Kühlmischung oder einer erfindungsgemäßen kosmetischen Zubereitung umfassend die Schritte:
a) Bereitstellen von 5-Methyl-2-(propan-2-yl)cyclohexyl-N-ethyloxamat (Formel I) 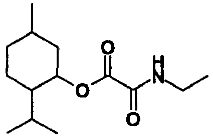
b) Bereitstellen eines, zweier, dreier oder mehrerer Polyole, ausgewählt aus der Gruppe A bestehend aus verzweigten oder unverzweigten Alkandiolen und verzweigten oder unverzweigten Alkantriolen mit jeweils 3-12 Kohlenstoffatomen und
c) Vermischen der in Schritt a) und b) bereitgestellten Komponenten.

### Beispiele

Nachfolgend wird die Erfindung anhand von Beispielen weiter verdeutlicht. Diese Beispiele dienen nicht dazu, die Erfindung einzuschränken. Sofern nicht anders angegeben beziehen sich alle Angaben auf das Gewicht.

### Anwendungsbeispiel

### Bewertung einer erfindungsgemäßen kosmetischen Zubereitung in vivo:

Ziel der humanen in vivo Studien war es, die Wirkung der erfindungsgemäßen Kühlmischung in O/W-Formulierungen auf Kühlintensität hin zu testen. Hierbei sollte geprüft werden, ob in der erfindungsgemäßen Wirkstoffkombination die Kühlwirkung/Kühlintensität von 5-Methyl-2-(propan-2-yl)cyclohexyl-N-ethyloxamat verstärkt wird. Jeder Einzeltest wurde mit Hilfe von 20 Panellisten unter standardisierten Bedingungen in einem Testinstitut durchgeführt. Hierzu hat jeder Proband zwei definierte Mengen von Cremeproben gleichzeitig auf beide Wangen verteilt. Dieser Halbseitentest erlaubt den direkten Vergleich von zwei Proben, wodurch die Reproduzierbarkeit der Ergebnisse deutlich höher ist als bei einem Versuch bei dem sich der Proband Kühlintensitäten merken muss. Nach Auftragung der Proben bewerteten die Probanden die Kühiintensität der zu vergleichenden Formulierungen auf einer Skala von 1-10 in einem Zeitraum von 30 Minuten (1=schwach; 10=stark). Die Studien wurden doppelt blind durchgeführt, hierzu erhielt das Testinstitut die Formulierungen in codierter Form. Um eine Standardisierung der Tests zu gewährleisten wurden folgende Bedingungen gewählt:
- Gleichmäßige Lüftungs- und Temperaturverhältnisse in dem Probandenraum
- Verwendung eines standardisierten Testprotokolls
- Detaillerte Erklärung des Testprotokolls
- Proben wurden den Probanden in einheitlichen, klaren Gefäßen übergeben
- Proben wurden über einen dreistelligen Code versehen
- Probenmenge von 0,3g Crème wurde auf jede Wange aufgetragen
- Proben wurden mit Deckeln versehen, um ein Austrocknen der Proben zu vermeiden
- Probanden wurden angehalten für den Tag des Tests kein Makeup sowie mindestens zwei Stunden vor dem Test keine kosmetischen Produkte zu verwenden
- Zu 32 identischen Zeitpunkten nach Anwendung haben die Probanden die Kühlwirkung der beiden Produkte auf einer Skala von 1-10 bewertet (1=schwach, 10=stark)

Die bei den Studien erhobenen Daten wurden hinsichtlich signifikanter Unterschiede zwischen den beiden Proben untersucht. Hierzu wurden die Daten zunächst auf Standardnormalverteilung hin untersucht. Sofern die Daten die Kriterien einer Standardnormalverteilung erfüllten wurde ein paarweiser t-test durchgeführt. Da jeder Proband ein unterschiedlich starkes Kühlempfinden hat und es sich bei dem Halbseitentest um abhängige Daten handelt, ist der gepaarte t-Test anzuwenden. Lag bei den Werten keine Normalverteilung vor, so wurde die Signifikanz über einen nichtparametrischen Wilcoxon überprüft. Die Formulierungen hatten die folgende Zusammensetzung:
**O/W Emulsion**

**Tabelle 1**

| | **Rohstoff** | **INCl** | **w/w %** | **w/w %** |
|---|---|---|---|---|
| | **(Lieferant)** | | **A** | **B** |
| **A.** | Emulsiphos^{®} (Symrise) | Potassium Cetyl Phosphate, Hydrogenated Palm Glycerides | 2,0 | 2,0 |
| | Cutina PES (Cognis) | Pentaerythrityl Distearate | 2,0 | 2,0 |
| | PCL Solid (Symrise) | Stearyl Heptanoate, Stearyl Caprylate | 2,0 | 2,0 |
| | Lanette O (Cognis) | Cetearyl Alcohol | 2,5 | 2,5 |
| | PCL Liquid 100 (Symrise) | Cetearyl Ethylhexanoate | 5,0 | 5,0 |
| | Isodragol^{®} | Triisononanoin | 2,0 | 2,0 |
| | Dow Corning 246 Fluid (Biesterfeld) | Cyclohexasiloxane, Cyclopentasiloxane | 2,0 | 2,0 |
| | Dragoxat^{®} 89 (Symrise) | Ethylhexyl Isononanoate | 3,0 | 3,0 |
| **B.** | Ultrez-10 (Noveon) | Carbomer | 0,2 | 0,2 |
| | Keltrol CG (CP Kelco) | Xanthan Gum | 0,15 | 0,15 |
| **C.** | Wasser | Water (Aqua) | ad 100 | ad 100 |
| | EDTA BD (BASF) | Disodium EDTA | 0,1 | 0,1 |
| | 1,2-Pentandiol (Dow) | 1,2-Pentandiol | 2,0 | - |
| **D.** | Sodium Hydroxide 10% sol. | Sodium Hydroxide | 0,2 | 0,2 |
| **E.** | 1,2-Pentandiol (Dow) | 1,2-Pentandiol | 3,0 | - |
| | MEO | (1*R*,2*S*,5*R*)-5-methyl-2-(propan-2-yl)cyclohexyl Nethyloxamate | 1,0 | 1,0 |
| | **pH- Wert** | | **6,0** | **6,0** |

### Herstellungshinweise:

Phasen A und C werden getrennt auf ca. 80°C erhitzt,. Phase B in Phase A dispergiert.

Phase C wird zu Phase AB gegeben und anschließend unter einem Ultra Turrax Rührer emulgiert (3 min). Anschließend wird Phase D zugegeben und neutralisiert. Die Emulsion wird mit einem Blattrührer kaltgerührt, wobei die Rührgeschwindigkeit mit abnehmender Temperatur reduziert wird.

Phase E wird bei 45-50°C zugegeben.

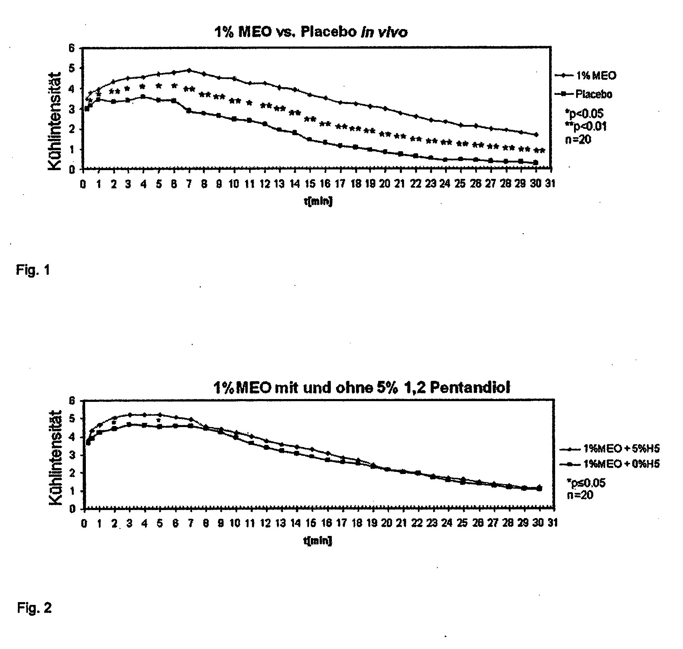

Die Figur 1 zeigt eine deutliche Kühlwirkung von (1*R*,2*S,*5*R*)-5-methyl-2-(propan-2-yl)cyclohexyl N ethyloxamat (MEO). Die Kühlwirkung ist unmittelbar nach Applikation der Formulierung spürbar. Des Weiteren ist die Dauer der Kühlwirkung mit über 30 Minuten äußerst ausgeprägt. Figur 2 zeigt die Wirkung einer erfindungsgemäßen Kühlmischung (Mischung A aus Tabelle 1), umfassend (1*R*,2*S*,5*R*)-5-methyl-2-(propan-2-yl)cyclohexyl_N-ethyloxamat und n-1,2-Pentandiol, verglichen mit einer Mischung identischer Zusammensetzung, bei der das n-1,2-Pentandiol durch Wasser ersetzt wurde (Mischung B aus Tabelle 1). Es zeigt sich, dass sich die Kühlwirkung der Pentandiol enthaltenden erfindungsgemäßen Kühlmischung durchgehend stärker bewertet wird. Nach zwei Minuten oder fünf Minuten ist die Steigung der Kühlintensität durch 1,2-Pentandiol auch mathematisch signifikant. Es ist davon auszugehen dass bei einem größeren Panel auch weitere signifikante Messwerte erzeugt werden können. Eine entsprechende Verstärkung der Wirkung von MEO sowie anderen Kühlstoffen lässt sich auch durch die anderen Diole und Triole erzeugen, die erfindungsgemäß Bestandteil der Gruppe b) der erfindungsgemäßen Kühlmischung sein können.

Fig. 1 dient insbesondere dazu, zu zeigen, dass MEO an sich schon eine Kühlwirkung ausübt, während Fig. 2 die Verstärkung dieser Kühlwirkung durch das erfindungsgemäß einzusetzende Diol belegt. Aufgrund des Messverfahrens (Wangentests mit jeweils nur zwei Vergleichswerten) sind zwei separate Versuchsreihen erforderlich gewesen, die sich in den zwei getrennten Figuren niederschlagen.

### Formulierungsbeispiele

### Formulierungsbeispiel 1A: Haarwasser

| | % | Inhaltsstoff |
|---|---|---|
| A | q.s. | Parfümöl |
| | 1,00 | PEG-40 Hydrogenated Castor Oil |
| B | 65,0 | Alkohol |
| | 1,0 | Panthenol |
| | 0,5 | Polyquarternium-16 |
| | 0,1 | Menthol |
| | 2,00 | (1*R*,2*S*,5*R*)-5-methyl-2-(propan-2-yl)cyclohexyl N ethyloxamat |
| | 3,00 | Pentylenglykol |
| | 27,4 | Aqua dem. |

Herstellung: Phase A mischen. Phase B zugeben und rühren bis alles gelöst ist, pH-Wert einstellen auf pH 7,0.

### Formulierungsbeispiel 1B: Haarwasser

| | % | Inhaltsstoff |
|---|---|---|
| A | q.s. | Parfümöl |
| | 1,00 | PEG-40 Hydrogenated Castor Oil |
| B | 65,0 | Alkohol |
| | 1,0 | Panthenol |
| | 0,5 | Polyquarternium-16 |
| | 0,1 | Menthol |
| | 2,00 | (1*R*,2*S*,5*R*)-5-methyl-2-(propan-2-yl)cyclohexyl N ethyloxamat |
| | 3,00 | Pentylenglykol |
| | 1,00 | 4-t Butylcyclohexanol |
| | 26,4 | Aqua dem. |

Herstellung: Phase A mischen. Phase B zugeben und rühren bis alles gelöst ist, pH-Wert einstellen auf pH 7,0.

### Formulierungsbeispiel 2A: Haargel

| | % | Inhaltsstoff |
|---|---|---|
| A | 45,00 | Carbopol 940 1 % in Wasser |
| | 0,70 | Aminomethyl Propanol |
| B | 7,50 | VP/Methacrylamide/Vinyl Imidazole Copolymer |
| | 0,10 | Parfümöl |
| | 0,30 | PEG-40 Hydrogenated Castor Oil |
| | 0,30 | Konservierungsmittel |
| | 0,05 | Disodium EDTA |
| | 0,30 | Panthenol |
| | 8,00 | Alkohol |
| | 5,00 | Pentylenglycol |
| | 2,00 | (1*R*,2*S*,5*R*)-5-methyl-2-(propan-2-yl)cyclohexyl N ethyloxamat |
| | 30,75 | Aqua dem. |

Herstellung: Phase A mischen. Phase B zugeben und rühren bis alles gelöst ist, pH-Wert einstellen auf pH 7,0.

### Formulierungsbeispiel 2B: Haargel

| | % | Inhaltsstoff |
|---|---|---|
| A | 45,00 | Carbopol 940 1% in Wasser |
| | 0,70 | Aminomethyl Propanol |
| B | 7,50 | VP/Methacrylamide/Vinyl Imidazole Copolymer |
| | 0,10 | Parfümöl |
| | 0,30 | PEG-40 Hydrogenated Castor Oil |
| | 0,30 | Konservierungsmittel |
| | 0,05 | Disodium EDTA . |
| | 0,30 | Panthenol |
| | 8,00 | Alkohol |
| | 5,00 | Pentylenglycol |
| | 0,5 | 4-t Butylcyclohexanol |
| | 1,50 | (1*R*,2*S*,5*R*)-5-methyl-2-(propan-2-yl)cyclohexyl N ethyloxamat |
| | 30,75 | Aqua dem. |

Herstellung: Phase A mischen. Phase B zugeben und rühren bis alles gelöst ist, pH-Wert einstellen auf pH 7,0.

### Formullerungsbeispiel 3A: Kosmetische Sonnenschutzzubereitung

In den folgenden Rezepturen wird eine kosmetische Sonnenschutzzubereitung, enthaltend eine Kombination aus mindestens anorganischem Pigment und organische m UV-Filter beschrieben.

Die Herstellung der nachfolgend genannten Formulierungen erfolgt auf übliche, dem Fachmann bekannte Art und Weise.

| | % | Inhaltsstoff |
|---|---|---|
| A | 7,50 | Ethylhexylzimtsäure |
| | 2,00 | Benzophenon-3 |
| | 0,80 | Polyglyceryldimersoyat |
| | 1,00 | Sorbitanstearat |
| | 0,50 | Tocopherylacetat |
| | 3,00 | Glycerylstearat, PEG-100 Stearat |
| | 1,00 | PEG-40-hydriertes Rizinusöl |
| B | 3,00 | Titandioxid, Aluminiumoxidhydrat, Dimethicon-/Methicon Copolymer |
| | 1,00 | *Butyrospermum parkii* (Shea Butter) |
| | 6,50 | C₁₂₋₁₅-Alkylbenzoat |
| C | 5,00 | Butylenglycol |
| | 0,30 | Xanthangummi |
| | 0,10 | Dinatrium-EDTA |
| | 0,10 | Allantoin |
| D | 1,00 | Polyacrylamid, C₁₃₋₁₄-Isoparaffin, Laureth-7 |
| | 5,00 | Pentylenglycol |
| | 2,00 | (1*R*,2*S*,5*R*)-5-methyl-2-(propan-2-yl)cyclohexyl N ethyloxamat |
| | 59,20 | Aqua dem. |

### Formulierungsbeispiel 3B: Kosmetische Sonnenschutzzubereitung

| | % | Inhaltsstoff |
|---|---|---|
| A | 7,50 | Ethylhexylzimtsäure |
| | 2,00 | Benzophenon-3 |
| | 0,80 | Polyglyceryldimersoyat |
| | 1,00 | Sorbitanstearat |
| | 0,50 | Tocopherylacetat |
| | 3,00 | Glycerylstearat, PEG-100 Stearat |
| | 1,00 | PEG-40-hydriertes Rizinusöl |
| B | 3,00 | Titandioxid, Aluminiumoxidhydrat, Dimethicon-/Methicon Copolymer |
| | 1,00 | *Butyrospermum parkii* (Shea Butter) |
| | 6,50 | C₁₂₋₁₅-Alkylbenzoat |
| C | 5,00 | Butylenglycol |
| | 0,30 | Xanthangummi |
| | 0,10 | Dinatrium-EDTA |
| | 0,10 | Allantoin |
| D | 1,00 | Polyacrylamid, C₁₃₋₁₄-Isoparaffin, Laureth-7 |
| | 5,00 | Pentylenglycol |
| | 1,00 | 4-t Butylcyclohexanol |
| | 2,00 | (1*R*,2*S*,5*R*)-5-methyl-2-(propan-2-yl)cyclohexyl N ethyloxamat |
| | 60,20 | Aqua dem. |

### Formulierungsbeispiel 4A: Feuchtigkeitsspendende Körperpflegecreme

| | % | Inhaltsstoff |
|---|---|---|
| A | 6,0 | PEG-7-hydriertes Rizinusöl |
| | 10,0 | Cetearylethylhexanoat |
| | 5,0 | Isopropylmyristat |
| | 7,0 | Mineralöl |
| | 0,5 | Shea Butter (*Butyrospermum parkii*) |
| | 0,5 | Aluminumstearat |
| | 0,5 | Magnesiumstearat |
| | 0,2 | Bisabolol |
| | 0,7 | Quaternium-18-Hectorit |
| B | 5,0 | Dipropylenglycol |
| | 0,7 | Magnesiumsulfat |
| | q.s. | Konservierungsmittel |
| | q.s. | Parfümöl |
| | 4,00 | Pentylenglycol |
| | 1,00 | (1*R*,2*S*,5*R*)-5-methyl-2-(propan-2-yl)cyclohexyl Nethyloxamat |
| | 58,9 | Aqua dem. |

Herstellung: Die Phasen A und B getrennt auf ca. 80 °C erwärmen. Phase B in Phase A einrühren und homogenisieren. Unter Rühren auf ca. 40 °C abkühlen, Phase C zugeben und nochmals homogenisieren. Unter Rühren auf Raumtemperatur abkühlen lassen.

### Formulierungsbeispiel 4B: Feuchtigkeitsspendende Körperpflegecreme

| | % | Inhaltsstoff |
|---|---|---|
| A | 6,0 | PEG-7-hydriertes Rizinusöl |
| | 10,0 | Cetearylethylhexanoat |
| | 5,0 | Isopropylmyristat |
| | 7,0 | Mineralöl |
| | 0,5 | Shea Butter (*Butyrospormum parkii*) |
| | 0,5 | Aluminumstearat |
| | 0,5 | Magnesiumstearat |
| | 0,2 | Bisabolol |
| | 0,7 | Quatemium-18-Hectorit |
| B | 5,0 | Dipropylenglycol |
| | 0,7 | Magnesiumsulfat |
| | q.s. | Konservierungsmittel |
| | q.s. | Parfümöl |
| | 4,00 | Pentylenglycol |
| | 1,00 | (1*R*,2*S*,5*R*)-5-methyl-2-(propan-2-yl)cyclohexyl Nethyloxamat |
| | 0,50 | 4-t Butylcyclohexanol |
| | 58,4 | Aqua dem. |

Herstellung: Die Phasen A und B getrennt auf ca. 80 °C erwärmen. Phase B in Phase A einrühren und homogenisieren. Unter Rühren auf ca. 40 °C abkühlen, Phase C zugeben und nochmals homogenisieren. Unter Rühren auf Raumtemperatur abkühlen lassen.

### Formulierungsbeisptel 5A: Pflegeshampoo

| | % | Inhaltsstoff |
|---|---|---|
| A | 30,0 | Natriumlaurethsulfat |
| | 6,0 | Natriumocoamphoacetat |
| | 6,0 | Cocamidopropylbetain |
| | 3,0 | Natriumlaurethsulfat, Glykoldistearat, Cocamid-MEA, Laureth-10 |
| | 7,2 | Polyquaternium-44 |
| | 2,0 | Amodimethicon |
| | q.s. | Parfümöl |
| | 1,0 | Natriumchlorid |
| B | 3,00 | Pentylenglycol |
| | 1,5 | (1*R*,2*S*,5*R*)-5-methyl-2-(propan-yl)cyclohexyl N ethyloxamat |
| | 40,3 | Aqua dem. |

Herstellung: Die Komponenten der Phase A mischen und lösen. Den pH-Wert mit Zitronensäure auf 6-7 einstellen.

### Formulferungsbeispiel 5B: Pflegeshampoo

| | % | Inhaltsstoff |
|---|---|---|
| A | 30,0 | Natriumlaurethsulfat |
| | 6,0 | Natriumocoamphoacetat |
| | 6,0 | Cocamidopropylbetain |
| | 3,0 | Natriumlaurethsulfat, Glykoldistearat, Cocamid-MEA, Laureth-10 |
| | 7,2 | Polyquatemium-44 |
| | 2,0 | Amodimethicon |
| | q.s. | Parfümöl |
| | 1,0 | Natriumchlorid |
| B | 2,50 | Pentylenglycol |
| | 1,5 | (1*R*,2*S*,5*R*)-5-methyl-2-(propan-2-yl)cyclohexyl N ethyloxamat |
| | 0,5 | 4-t Butylcyclohexanol |
| | 40,3 | Aqua dem. |

Herstellung: Die Komponenten der Phase A mischen und lösen. Den pH-Wert mit Zitronensäure auf 6-7 einstellen.

### Formulierungsbeisplel 6A: Duschgel

| | % | Inhaltsstoff |
|---|---|---|
| A | 40,0 | Natriumlaurethsulfat |
| | 5,0 | Decylglukosid |
| | 5,0 | Cocamidopropylbetain |
| | 1,0 | (1*R*,2*S*,5*R*)-5-methyl-2-(propan-2-yl)cyclohexyl N ethyloxamat |
| | 1,0 | Panthenol |
| | q.s. | Parfümöl |
| | 3,0 | Pentylenglycol |
| | 2,0 | Natriumchlorid |
| | 43,0 | Aqua dem. |
| B | q.s. | Zitronensäure |

Herstellung: Die Komponenten der Phase A mischen und lösen. Den pH-Wert mit Zitronensäure auf 6-7 einstellen.

### Formulierungsbeispiel 6B: Duschgel

| | % | Inhaltsstoff |
|---|---|---|
| A | 40,0 | Natriumlaurethsulfat |
| | 5,0 | Decylglukosid |
| | 5,0 | Cocamidopropylbetain |
| | 1,0 | (1*R*,2*S*,5*R*)-5-methyl-2-(propan-2-yl)cyclohexyl N ethyloxamat |
| | 1,0 | Panthenol |
| | q.s. | Parfümöl |
| | 3,0 | Pentylenglycol |
| | 2,0 | Natriumchlorid |
| | 42,6 | Aqua dem. |
| | 0,4 | 4-t Butylcyclohexanol |
| B | q.s. | Zitronensäure |

Herstellung: Die Komponenten der Phase A mischen und lösen. Den pH-Wert mit Zitronensäure auf 6-7 einstellen.

### Formulierungsbeispiel 7A: Shampoo

| | | |
|---|---|---|
| | % | Inhaltsstoff |
| A | 40,0 | Natriumlaurethsulfat |
| | 5,0 | Natrium-C₁₂₋₁₅-Pareth-15-sulfonat |
| | 5,0 | Decylglukosid |
| | q.s. | Parfümöl |
| | 0,1 | Phytantriol |
| | 40,6 | Aqua dem. |
| | 0,5 | (1*R*,2*S*,5*R*)-5-methyl-2-(propan-2-yl)cyclohexyl N ethyloxamat |
| | 0,3 | Polyquatemium-10 |
| | 1,0 | Panthenol |
| | q.s. | Konservierungsmittel |
| | 4,5 | Pentylenglycol |
| | 1,0 | Laureth-3 |
| | 2,0 | Natriumchlorid |

Herstellung: Die Komponenten der Phase A mischen und lösen. Den pH-Wert mit Zitronensäure auf 6-7 einstellen.

### Formulierungsbeisplel 7B: Shampoo

| | % | Inhaltsstoff |
|---|---|---|
| A | 40,0 | Natriumlaurethsulfat |
| | 5,0 | Natrium-C₁₂₋₁₅-Pareth-15-sulfonat |
| | 5,0 | Decylglukosid |
| | q.s. | Parfümöl |
| | 0,1 | Phytantriol |
| | 40,3 | Aqua dem. |
| | 0,5 | (1*R*,2*S*,5*R*)-5-methyl-2-(propan-2-yl)cyclohexyl N ethyloxamat |
| | 0,3 | Polyquatemium-10 |
| | 1,0 | Panthenol |
| | q.s. | Konservierungsmittel |
| | 4,5 | Pentylenglycol |
| | 1,0 | Laureth-3 |
| | 0,3 | 4-t Butylcyclohexanol |
| | 2,0 | Natriumchlorid |

Herstellung: Die Komponenten der Phase A mischen und lösen. Den pH-Wert mit Zitronensäure auf 6-7 einstellen.

### Formulierungsbeisplel 8A: Fußbalsam

| | % | Inhaltsstoff |
|---|---|---|
| A | 2,0 | Ceteareth-6, Stearylalkohol |
| | 2,0 | Ceteareth-25 |
| | 5,0 | Cetearylethylhexanoat |
| | 4,0 | Cetylalkohol |
| | 4,0 | Glycerylstearat |
| | 5,0 | Mineralöl |
| | 0,2 | Menthol |
| | 0,5 | Kampfer |
| B | 65,3 | Aqua dem. |
| | q.s. | Konservierungsmittel |
| C | 1,0 | Bisabolol |
| | 1,0 | Tocopherylacetat |
| D | 0,5 | (1*R*,2*S*,5*R*)-5-methyl-2-(propan-2-yl)cyclohexyl N ethyloxamat |
| | 4,5 | Pentylenglycol |
| | 5,0 | Zaubernussextrakt |

Herstellung: Die Komponenten der Phasen A und B getrennt voneinander auf ca. 80 °C erwärmen. Phase B in Phase A unter Homogenisieren einrühren. Unter Rühren abkühlen auf ca. 40 °C, die Phasen C und D hinzugeben und kurz nachhomogenisieren. Unter Rühren auf Raumtemperatur abkühlen.

### Formullerungsbeisplel 8B: Fußbalsam

| | % | Inhaltsstoff |
|---|---|---|
| A | 2,0 | Ceteareth-6, Stearylalkohol |
| | 2,0 | Ceteareth-25 |
| | 5,0 | Cetearylethylhexanoat |
| | 4,0 | Cetylalkohol |
| | 4,0 | Glycerylstearat |
| | 5,0 | Mineralöl |
| | 0,2 | Menthol |
| | 0,5 | Kampfer |
| B | 65,3 | Aqua dem. |
| | q.s. | Konservierungsmittel |
| C | 1,0 | Bisabolol |
| | 1,0 | Tocopherylacetat |
| D | 0,5 | (1*R*,2*S*,5*R*)-5-methyl-2-(propan-2-yl)cyclohexyl N ethyloxamat |
| | 4,5 | Pentylenglycol |
| | 0,3 | 4-t Butylcyclohexanol |
| | 4,7 | Zaubemussextrakt |

Herstellung: Die Komponenten der Phasen A und B getrennt voneinander auf ca. 80 °C erwärmen. Phase B in Phase A unter Homogenisieren einrühren. Unter Rühren abkühlen auf ca. 40 °C, die Phasen C und D hinzugeben und kurz nachhomogenisieren. Unter Rühren auf Raumtemperatur abkühlen.

### Formulierungsbeispiel 9A: Gesichtsreinigungslotion - Typ O/W

| | % | Inhaltsstoff |
|---|---|---|
| A | 10,0 | Cetearylethylhexanoat |
| | 10,0 | Capryl-/Caprintriglycerid |
| | 1,5 | Cyclopentasiloxan, Cyclohexasilosan |
| | 2,0 | PEG-40-hydriertes Rizinusöl |
| B | 3,5 | Capryl-/Caprintriglycerid, Natriumacrylat-Copolymer |
| C | 1,0 | Tocopherylacetat |
| | 0,2 | Bisabolol |
| | q.s. | Konservierungsmittel |
| | q.s. | Parfümöl |
| D | 3,0 | Polyquaternium-44 |
| | 0,5 | Cocotrimoniummethosulfat |
| | 0,5 | Ceteareth-25 |
| | 2,0 | Panthenol, Propylenglycol |
| | 4,0 | Pentylenglycol |
| | 0,1 | Dinatrium-EDTA |
| | 1,0 | (1*R*,2*S*,5*R*)-5-methyl-2-(propan-2-yl)cyclohexyl N ethyloxamat |
| | 60,7 | Aqua dem. |

Herstellung: Phase A lösen. Phase B in Phase A einrühren, Phase C in die kombinierten Phasen A und B einarbeiten. Phase D lösen, in die kombinierten Phasen A, B und C einrühren und homogenisieren. 15 min nachrühren.

### Formulierungsbeispiel 9B: Gesichtsreinigungslotion - Typ O/W

| | % | Inhaltsstoff |
|---|---|---|
| A | 10,0 | Cetearylethylhexanoat |
| | 10,0 | Capryl-/Caprintriglycerid |
| | 1,5 | Cyclopentasiloxan, Cyclohexasilosan |
| | 2,0 | PEG-40-hydriertes Rizinusöl |
| B | 3,5 | Capryl-/Caprintriglycerid, Natriumacrylat-Copolymer |
| C | 1,0 | Tocopherylacetat |
| | 0,2 | Bisabolol |
| | q.s. | Konservierungsmittel |
| | q.s. | Parfümöl |
| D | 3,0 | Polyquaternium-44 |
| | 0,5 | Cocotrimoniummethosulfat |
| | 0,5 | Ceteareth-25 |
| | 2,0 | Panthenol, Propylenglycol |
| | 4,0 | Pentylenglycol |
| | 0,1 | Dinatrium-EDTA |
| | 1,0 | (1*R*,2*S*,5*R*)-5-methyl-2-(propan-2-yl)cyclohexyl N ethyloxamat |
| | 1,0 | 4-t-Butylcyclohexanol |
| | 59,7 | Aqua dem. |

Herstellung: Phase A lösen. Phase B in Phase A einrühren, Phase C in die kombinierten Phasen A und B einarbeiten. Phase D lösen, in die kombinierten Phasen A, B und C einrühren und homogenisieren. 15 min nachrühren.

### Formulierungsbeispiel 10A: Body-Spray

| | % | Inhaltsstoff |
|---|---|---|
| A | 3,0 | Ethylhexylmethoxycinnamat |
| | 2,0 | Diethylamlnohydroxybenzoylhexylbenzoat |
| | 1,0 | Polyquaternium-44 |
| | 3,0 | Pentylenglycol |
| | 2,0 | Panthenol, Propylenglycol |
| | 1,0 | Cyclopentasiloxan, Cyclohexasilosan |
| | 10,0 | Octyldodecanol |
| | 0,5 | PVP |
| | 10,0 | Capryl-/Caprintriglycerid |
| | 3,0 | C₁₂₋₁₅-Alkylbenzoat |
| | 3,0 | Glycerin |
| | 1,0 | Tocopherylacetat |
| | 0,3 | Bisabolol |
| | 0,5 | (1*R*,2*S*,5*R*)-5-methyl-2-(propan-2-yl)cyclohexyl N ethyloxamat |
| | 59,7 | Alkohol |

Herstellung: Die Komponenten der Phase A einwiegen und klar lösen.

### Formulierungsbeispiel 10B: Body-Spray

| | % | Inhaltsstoff |
|---|---|---|
| A | 3,0 | Ethylhexylmethoxycinnamat |
| | 2,0 | Diethylaminohydroxybenzoylhexylbenzoat |
| | 1,0 | Polyquaternium-44 |
| | 3,0 | Pentylenglycol |
| | 2,0 | Panthenol, Propylenglycol |
| | 1,0 | Cyclopentasiloxan, Cyclohexasilosan |
| | 10,0 | Octyldodecanol |
| | 0,5 | PVP |
| | 10,0 | Capryl-/Caprintriglycerid |
| | 3,0 | C₁₂₋₁₆-Alkylbenzoat |
| | 3,0 | Glycerin |
| | 1,0 | Tocopherylacetat |
| | 0,3 | Bisabolol |
| | 0,5 | (1*R*,2*S*,5*R*)-5-methyl-2-(propan-2-yl)cyclohexyl N ethyloxamat |
| | 0,5 | 4-t-Butylcyclohexanol |
| | 59,2 | Alkohol |

Herstellung: Die Komponenten der Phase A einwiegen und klar lösen.

### Formulierungsbeispiel 11A: Hautpflegegel

| | % | Inhaltsstoff |
|---|---|---|
| A | 3,6 | PEG-40-hydriertes Rizinusöl |
| | 15,0 | Alkohol |
| | 0,1 | Bisabolol |
| | 0,5 | Tocopherylacetat |
| | q.s. | Parfümöl |
| B | 3,0 | Panthenol |
| | 0,6 | Carbomer |
| | 4,0 | Pentylenglycol |
| | 0,5 | (1*R*,2*S*,5*R*)-5-methyl-2-(propan-2-yl)cyclohexyl N ethyloxamat |
| | 71,9 | Aqua dem. |
| C | 0,8 | Triethanolamin |

### Formulierungsbeispiel 11B: Hautpflegegel

| | % | Inhaltsstoff |
|---|---|---|
| A | 3,6 | PEG-40-hydriertes Rizinusöl |
| | 15,0 | Alkohol |
| | 0,1 | Bisabolol |
| | 0,5 | Tocopherylacetat |
| | q.s. | Parfümöl |
| B | 3,0 | Panthenol |
| | 0,6 | Carbomer |
| | 4,0 | Pentylenglycol |
| | 0,5 | (1*R*,2*S*,5*R*)-5-methyl-2-(propan-2-yl)cyclohexyl N ethyloxamat |
| | 71,7 | Aqua dem. |
| | 0,2 | 4-t-Butylcyclohexanol |
| C | 0,8 | Triethanolamin |

### Formulierungsbeisplel 12A: After-Shave-Lotion

| | % | Inhaltsstoff |
|---|---|---|
| A | 10,0 | Cetearylethylhexanoat |
| | 5,0 | Tocopherylacetat |
| | 1,0 | Bisabolol |
| | 0,1 | Parfümöl |
| | 0,3 | Acrylate/C₁₀₋₃₀ Alkylacrylat-Crosspolymer |
| B | 15,0 | Alkohol |
| | 1,0 | Panthenol |
| | 3,0 | Glycerin |
| | 1,0 | (1*R*,2*S*,5*R*)-5-methyl-2-(propan-2-yl)cyclohexyl N ethyloxamat |
| | 4,0 | Pentylenglycol |
| | 0,1 | Triethanolamin |
| | 59,5 | Aqua dem. |

Herstellung: Die Komponenten der Phase A mischen. Phase B lösen, in Phase A einarbeiten und homogenisieren.

### Formullerungsbelspilel 12B: After-Shave-Lotion

| | % | Inhaltsstoff |
|---|---|---|
| A | 10,0 | Cetearylethylhexanoat |
| | 5,0 | Tocopherylacetat |
| | 1,0 | Bisabolol |
| | 0,1 | Parfümöl |
| | 0,3 | Acrylate/C₁₀₋₃₀ Alkylacrylat-Crosspolymer |
| B | 15,0 | Alkohol |
| | 1,0 | Panthenol |
| | 3,0 | Glycerin |
| | 1,0 | (1*R*,2*S*,5*R*)-5-methyl-2-(propan-2-yl)cyclohexyl N ethyloxamat |
| | 4,0 | Pentylenglycol |
| | 0,1 | Triethanolamin |
| | 0,6 | 4-t-Butylcyclohexanol |
| | 58,9 | Aqua dem. |

Herstellung: Die Komponenten der Phase A mischen. Phase B lösen, in Phase A einarbeiten und homogenisieren.

### Formulierungsbeispiel 13A: After-Sun-Lotion

| | % | Inhaltsstoff. |
|---|---|---|
| A | 0,4 | Acrylate/C 10-30-Alkylacrylat-Crosspolymer |
| | 15,0 | Cetearylethylhexanoat |
| | 0,2 | Bisabolol |
| | 1,0 | Tocopherylacetat |
| | q.s. | Parfümöl |
| B | 1,0 | Panthenol |
| | 15,0 | Alkohol |
| | 3,0 | Glycerin |
| | 1,5 | (1*R*,2*S*,5*R*)-5-methyl-2-(propan-2-yl)cyclohexyl N ethyloxamat |
| | 4,0 | Pentylenglycol |
| | 58,7 | Aqua dem. |
| C | 0,2 | Triethanolamin |

Herstellung: Die Komponenten der Phase A mischen. Phase B unter Homogenisieren in Phase A einrühren. Mit Phase C neutralisieren und erneut homogenisieren.

### Formulierungsbeispeil 13B: After-Sun-Lotion

| | % | Inhaltsstoff |
|---|---|---|
| A | 0,4 | Acrylate/C 10-30-Alkylacrylat-Crosspolymer |
| | 15,0 | Cetearylethylhexanoat |
| | 0,2 | Bisabolol |
| | 1,0 | Tocopherylacetat |
| | q.s. | Parfümöl |
| B | 1,0 | Panthenol |
| | 15,0 | Alkohol |
| | 3,0 | Glycerin |
| | 1,5 | (1*R*,2*S*,5*R*)-5-methyl-2-(propan-2-yl)cyclohexyl N ethyloxamat |
| | 4,0 | Pentylengllycol |
| | 2,0 | 4-t-Butylcyclohexanol |
| | 56,7 | Aqua dem. |
| C | 0,2 | Triethanolamin |

Herstellung: Die Komponenten der Phase A mischen. Phase B unter Homogenisieren in Phase A einrühren. Mit Phase C neutralisieren und erneut homogenisieren.

### Formulierungsbeispiel 14A: Sonnenschutzlotion

| | % | Inhaltsstoff |
|---|---|---|
| A | 4,5 | Ethylhexylmethoxyzimtsäure |
| | 2,0 | Diethylaminohydroxybenzoylhexylbenzoat |
| | 3,0 | Octocrylen |
| | 2,5 | Di-C12-13-Alkylmalat |
| | 0,5 | Tocopherylacetat |
| | 4,0 | Polyglyceryl-3-methylglucosedistearat |
| B | 3,5 | Cetearylisononanoat |
| | 1,0 | VP-/EicosenCopolymer |
| | 5,0 | Isohexadecan |
| | 2,5 | Di-C12-13-Alkylmalat |
| | 3,0 | Titaniumdioxid, Trimethoxycaprylylsilan |
| C | 5,0 | Glycerin |
| | 1,0 | Natriumcetearylsulfat |
| | 0,5 | Xanthangummi |
| | 55,7 | Aqua dem. |
| D | 1,0 | (1*R*,2*S*,5*R*)-5-methyl-2-(propan-2-yl)cyclohexyl N ethyloxamat |
| | 4,0 | Pentylenglycol |
| | 1,0 | Phenoxyethanol, Methylparaben, Ethylparaben, Butylparaben, Propyl-paraben, Isobutylparaben |
| | 0,3 | Bisabolol |

Herstellung: Die Komponenten der Phasen A und B getrennt voneinander auf ca. 80 °C erwärmen. Phase B in Phase A einrühren und homogenisieren. Phase C auf ca. 80 °C erwärmen und unter Homogenisieren in die kombinierten Phasen A und B einrühren. Unter Rühren auf ca. 40 °C abkühlen, Phase D zugeben und nochmals homogenisieren.

### Formulierungsbeispiel 14B: Sonnenschutzlotion

| | % | Inhaltsstoff |
|---|---|---|
| A | 4,5 | Ethylhexylmethoxyzimtsäure |
| | 2,0 | Diethylaminohydroxybenzoylhexylbenzoat |
| | 3,0 | Octocrylen |
| | 2,5 | Di-C12-13-Alkylmalat |
| | 0,5 | Tocopherylacetat |
| | 4,0 | Polyglyceryl-3-methylglucosedistearat |
| B | 3,5 | Cetearylisonononoat |
| | 1,0 | VP-/EicosenCopolymer |
| | 5,0 | Isohexadecan |
| | 2,5 | Di-C12-13-Alkylmalat |
| | 3,0 | Titaniumdioxid, Trimethoxycaprylylsilan |
| C | 5,0 | Glycerin |
| | 1,0 | Natriumcetearylsulfat |
| | 0,5 | Xanthangummi |
| | 55,4 | Aqua dem. |
| D | 1,0 | (1*R*,2*S*,5*R*)-5-methyl-2-(propan-2-yl)cyclohexyl N ethyloxamat |
| | 4,0 | Pentylenglycol |
| | 0,3 | 4-t-Butylcyclohexanol |
| | 1,0 | Phenoxyethanol, Methylparaben, Ethylparaben, Butylparaben, Propyl-paraben, Isobutylparaben |
| | 0,3 | Bisabolol |

Herstellung: Die Komponenten der Phasen A und B getrennt voneinander auf ca. 80 °C erwärmen. Phase B in Phase A einrühren und homogenisieren. Phase C auf ca. 80 °C erwärmen und unter Homogenisieren in die kombinierten Phasen A und B einrühren. Unter Rühren auf ca. 40 °C abkühlen, Phase D zugeben und nochmals homogenisieren.

Die folgenden Bespiele verdeutlichen Einsatzmöglichkeiten der erfindungsgemäß zu verwendenden Kühlsubstanzen in kosmetischen Formulierungen, durch deren Verwendung auf der Haut ein als besonders angenehm empfundenes Kälteempfinden und eine Hautberuhigung erzielt werden können.

| | |
|---|---|
| **15 = Aerosol Deo-Spray** | **22 = Tagescreme O/W. ca. SPF 15** |
| **16 = Duschgel** | **23 = Sonnenschutzlotion ca. SPF 25** |
| **17 = After Shave Balsam** | **24 = After Sun Spray** |
| **18 = Eau de Toilette** | **25 = After Shave** |
| **19 = Fußspray** | **26 = Creme W/O** |
| **20 = Deo-Stick** | **27 = Haar Conditioner** |
| **21 = Deo APP Roll on Emulsion** | |

| | **INCI**-**Name** | **% w/w** | | | | | | | | | | | | |
|---|---|---|---|---|---|---|---|---|---|---|---|---|---|---|
| **Formulierungsbeispiel** | | **15** | **16** | **17** | **18** | **19** | **20** | **21** | **22** | **23** | **24** | **25** | **26** | **27** |
| **Inhaltsstoff** | | | | | | | | | | | | | | |
| (1R,2S,5R)-5-methyl-2-(propan-2-yl)cyclohexyl N ethyloxamat | | 0.5 | 0.5 | 0.5 | 0.5 | 0.5 | 0.5 | 0.5 | 0.5 | 0.5 | 0.5 | 0.5 | 0.5 | 0.5 |
| SymSitive 1609 | Trans-4-tert-butyl cyclohexanol Pentylene Glycol | 1 | 1 | 1 | 1 | 1 | 1 | 1 | 1 | 1 | 1 | 1 | 1 | 1 |
| Allantoin | Allantoin | | | 0.1 | | | | | | | 0.1 | | | |
| (-) alpha Bisabolol natürlich | Bisabolol | 0.1 | | | | | | | | | 0.2 | | 0.3 | |
| Abil 350 | Dimethicone | | | 3.0 | | | | | 2.0 | | | | | |
| Akyposoft 100 BVC | Sodium Laureth-11 Carboxylate. Laureth-10 | | 8.5 | | | | | | | | | | | |
| Aloe Vera Gel Konzentrat 10:1 | Aloe Barbadensis Leaf Juice | | | | | | 1.0 | | | | | | | |
| Aluminiumstearat | Aluminium Stearate | | | | | | | | | | | | 1.2 | |
| F | Laureth-2 | | 2.5 | | | | | | | | | | | |
| Biotive® L-Arginine | Arginine | | | | | | | | | 0.5 | | | | |
| Carbopol Ultrez- 10 | Carbomer | | | | | | | | 0.2 | | 0.2 | | | |
| Carbopol Ultrez-21 | Acrylates/C10-30 Alkyl Acrylate Crosspolymer | | | 0.4 | | | | | | | | | | |
| Covi-Ox T-70 | Tocopherol | | | 0.1 | | | | | | | | 0.1 | | |
| Cutina GMS V | Glyceryl Stearate | | | | | | | | 2.0 | | 2.0 | | | |
| Dehyton K | Cocoamidopropyl Betaine | | 7.0 | | | | | | | | | | | |
| Dehyquart A CA | Cetrimonium Chloride | | | | | | | | | | | | | 4.0 |
| Deolite | Dimethyl Phenylpropanol Pentylene Glycol | | | | | | 0.5 | 0.5 | | | | | | |
| Dow Corning 246 fluid | Cyclohexasiloxane | | | | | | | 1.0 | | | | 2.0 | | |
| D-Panthenol 75 L | Panthenol | | | 1.0 | | | | | | | | 1.0 | | 1.0 |
| Dracorin® 100 S.E.P. | Glyceryl Stearate. PEG-100 Stearate | | | | | | | 0.5 | | | | | | |
| Dracorin® CE | Glyceryl Stearate/Citrate | | | | | | | | | 2.0 | | | | |
| Dracorin® GOC | Glyceryl Oleate Citrate Caprylic Capric Triglyceride | | | | | | | 2.0 | | | | 2.0 | | |
| Drago-Beta-Glucan | Water (Aqua). Butylene Glycol. Glycerin. Avena Sativa (Oat) Kernel Extract | | | | | | | | | | 2.0 | | | |
| DragoCalm® | Water. Glycerin. Avena Sativa (Oat Kernel Extract) | | | | | | | | | | 1.0 | | | |
| Dragocide® Liquid | Phenoxyethanol. Methylparaben. Ethylparaben. Butylparaben. Propylparaben. | | 0.5 | 0.8 | | | | 0.8 | 0.8 | | | | 0.8 | 0.8 |
| | Isobutylparaben | | | | | | | | | | | | | |
| Dragoderm® | Glycerin. Triticum Vulgare (Wheat) Gluten. Water (Aqua) | | | | | | | | | | 2.0 | 2.0 | | 2.0 |
| Dragosan W/O P | Sorbitan Isostearate. Hydrogenated Castor Oil. Ceresin. Beeswax (Cera Alba) | | | | | | | | | | | | 8.0 | |
| Dragosantol® 100 | Bisabolol | | | 0.2 | | 0.2 | | 0.2 | | | | | | |
| Dragosine® | Camosine | | | | | | | | | | 0.2 | | | |
| Dragoxat® 89 | Ethylhexyl Isononanoate | | | | | | 1.0 | | | 3.0 | 4.0 | 1.0 | 5.0 | |
| EDTABD | Disodium EDTA | | | 0.1 | | | | | | 0.1 | 0.1 | | | |
| Emulsiphos® | Potassium Cetyl Phosphate. Hydrogenated Palm Glycerides | | | | | | | | 2.0 | | 2.0 | | | |
| Ethanol 96% | Ethanol | 26 | | | 81.0 | 45.0 | | | | | | 65.0 | | |
| Extrapone®Ginkgo Biloba | Propylene Glycol. Water (Aqua). Ginkgo Biloba Leaf Extract. Glucose. Lactic Acid | | 1.0 | | | | | | | | | | | |
| Farnesol | Farnesol | | | | | 0.5 | | | | | | | | |
| Riechstoff | Perfum | 1.0 | 1.5 | 1.0 | 10.0 | 0.5 | 0.5 | 0.4 | 0.3 | 0.4 | 0.3 | 1.0 | 0.3 | 0.3 |
| Frescolat® MGA | Menthone Glycerin Acetal | | | | | | 1.0 | | | | | | | |
| Frescolat® ML | Menthyl Lactate | | 0.6 | 1.0 | | 0.3 | | 0.5 | 0.3 | | | 0.5 | | |
| Fruitapone® Orange B | Propylene Glycol. Water (Aqua). Citric Acid. Citrus Aurantium Dulcis (Orange) Juice. Trideceth-9. Bisabolol | | | | | | | | 1.0 | | | | | |
| Genapol LRO Liquid | Sodium Laureth Sulfate | | 40.0 | | | | | | | | | | | |
| Glycerin 99.5% | Glycerin | | | 2.5 | | | | 4.0 | 2.0 | | 3.0 | 4.0 | 3.0 | |
| Hydrolite®-5 | Pentylene Glycol | | | | | | | | | 5.0 | | 5.0 | | |
| Hydroviton®-24 | Water. Pentylene Glycol. Glycerin. Lactic Acid. Sodium Lactate. Serine. Urea. Sorbitol. Sodium Chloride. Allantoin | | | | | | | | | | 1.0 | | 2.0 | |
| Iso Adipat | Diisopropyl Adipate | | | | | | | | | | 1.0 | 5.0 | | |
| Isodragol® | Triisononanoin | | | | | | | 1.0 | | | | | | |
| Jojobaöl | Simmondsia Chinensis (Jojoba) Seed Oil | | | 2.0 | | | | | | | | | 2.0 | |
| Keltrol CG RD | Xanthan Gum | | | | | | | | 0.1 | 0.1 | 0.2 | | | |
| Lanette O | Cetearyl Alcohol | | | | | | | | 3.0 | 2.0 | 3.0 | | | 3.5 |
| Mineralöl | Mineral Oil | | | | | | | | | | | | 8.0 | |
| Natriumchlorid | Sodium Chloride | | | | | | | | | | | | 1.0 | 2.0 |
| Natriumhydroxid 10% Lsg. | Sodium Hydroxide | | 0.1 | 0.8 | | | | 0.6 | 0.5 | | | | 0.4 | |
| Natriumstearat | Sodium Stearate | | | | | | 9.0 | | | | | | | |
| Neo Heliopan® 303 | Octocrylene | | | | | | | | 5.0 | 8.0 | | | | |
| Neo Heliopan® 357 | Butylmethoxydibenz oylmethane | | | | | | | | 1.1 | 3.0 | | | | |
| Neo Heliopan® HMS | Homosalate | | | | | | | | | 5.0 | | | | |
| Neo Heliopan® Hydro. 25% Lsg. neutralisiert mit Biotive L-Arginin | Phenylbenzimidazole Sulfonic Acid | | | | | | | | 3.0 | 8.0 | | | | |
| Neo Heliopan®AP. 10% Lsg.. neutralisiert mit NAOH | Disodium Phenyl Dibenzimidazole Tetrasulfonate | | | | | | | | 3.0 | 133 | | | | |
| Neo Heliopan® OS | Ethylhexyl Salicylate | | | | | | | | 5.0 | | | | | |
| Neutralöl | Caprylic/Capric Triglyceride | | | | | | | 3.5 | | | | 5.0 | | |
| Ozokerite Wax 2389 | Ozokerite | | | | | | | | | | | | 2.0 | |
| PCL-Liquid100 | Cetearyl Ethylhexanoate | | | 3.0 | | 1.0 | | | | | | | | |
| Pemulen TR-2 | Acrylates/C10-30 Alkyl Acrylate Crosspolymer | | | | | | | 0.3 | | | | 0.3 | | |
| Polymer JR400 | Polyquaternium-10 | | 0.3 | | | | | | | | | | | |
| Polyquart H81 | PEG-15 Coco Polyamine | | | | | | | | | | | | | 3.0 |
| Propane Butane 2.7 bar | Propane. Butane | 70.4 | | | | 48 | | | | | | | | |
| Propylenglykol | Propylene Glycol | | | | | | 36.5 | | 3.0 | 4.0 | | | | |
| Rezal 36 GP | Aluminium Zirconium Tetrachlorohydrex GLY | | | | | | | 5.0 | | | | | | |
| Softisan 100 | Hydrogenated Coco Glycerides | | | | | | | | | 1.5 | | | | |
| Lösungsvermittler | PEG-40Hydrogenated Castor Oil. Trideceth-9. Propylene Glycol. Water (Aqua) | | 0.5 | | 1.0 | 1.0 | | | | | | | | |
| Squalan plfanzlich | Squalane | | | | | | | | | | 3.0 | | | |
| SymAmide UDA | Undecylenamide DEA. Diethanolamine | | | | | 1.0 | | | | | | | | |
| SymCalmin® | Pentylene Glycol. Butylene Glycol. Hydroxyphenyl Propamidobenzoic Acid | | | 0.5 | | | | | | | 1.0 | | | |
| SymClariol® | Decylene Glycol | 0.5 | | | | 0.5 | | | | | | | | |
| SymDeo® MPP | Dimethyl Phenylbutanol | 0.5 | | | | | | 0.5 | | | | | | |
| SymDiol® 68 | 1.2 Hexanediol. Caprylyl Glycol | | | | | | | | | | 1.0 | | | |
| SymGlucan® | Water (Aqua) Glycerin. Beta Glucan | | | | | | | | | | | 1.0 | | |
| SymMollient® W/S | Trideceth-9. PEG-5 Isononanoate | | | | 1.0 | 0.5 | | | | | | 0.5 | | |
| SymRelief® | Bisabolol. Zingiber Officinale (Ginger) Root Extract | | 0.2 | 0.2 | | | | | | | | 0.2 | | |
| SymRepair® | Hexyldecanol. Bisabolol. Cetylhydroxyproline Palmitamide. Stearic Acid. Brassica Campestris (Rapeseed Sterols) | | | | | | | | | | 2.0 | 3.0 | | |
| SymVital® | Aloe Barbadensis Leaf Juice Powder. Magnesium Ascorbyl Phosphate. Rubus Idaeus (Raspberry) Leaf Extract | | | 0.1 | | | | | 0.3 | | | | | |
| Triethanolamin 99% | Triethanolamine | | | | | | | | | | 0.4 | 0.3 | | |
| Vitamin E Acetat | Tocopherol Acetate | | | 0.5 | | | | | | 0.5 | | | 0.2 | |
| Wasser | Water (Aqua) | | ad100 | ad100 | ad100 | | ad100 | ad100 | ad100 | ad100 | ad100 | ad100 | ad100 | ad100 |

In sämtlichen Formulierungsbeispielen war die von Testpersonen empfundene Kühlwirkung gegenüber vergleichbaren Formulierungen, die frei waren von C3 - C12 Alkandiolen und Alkantriolen, verstärkt. Sofern die entsprechenden Formulierungen außerdem noch *trans*- 4-tert.Butylcyclohexanol enthielten, war die Kühlwirkung nochmals verglichen mit Formulierungen ohne diese Verbindung verstärkt.

Es hat sich ferner in der Praxis herausgestellt, dass vergleichbare Effekte erzielt werden, wenn in jeder dieser oben genannten Formulierungen der Anteil von 1,2-Pentandiol durch 1,2-Hexandiol im Verhältnis 5:3 ersetzt wird.

Das gleiche gilt für den Austausch von 1,2-Pentandiol durch 1,2-Oktandiol.

Analoges gilt auch für den Austauch von 1,2-Pentandiol durch 1,2-Dekandiol wobei letzteres nur in der Menge von einem Fünftel des Pentandiols eingesetzt wird.

Nach der vorliegenden Erfindung ist darüber hinaus zu erwarten, dass durch den Austausch von 5-Methyl-2-(propan-2-yl)cyclohexyl-N-ethyloxamat durch andere Oxamate oder andere Kühlstoffe, wie z. B. 5-Methyl-2-(propan-2-yl)cyclohexyl-N-methyloxamat ein ähnlicher Effekt erreicht werden kann.

Die Breite der Formulierung und Verschiedenheit der Beispiele zeigt, dass der verstärkende Kühleffekt im gesamten Bereich der Kosmetik erzielt werden kann. Dem Fachmann sind damit eine Vielzahl von zusätzlichen Kühlmischungen und Formulierungen zugänglich.

## Patentansprüche

1. Kühlmischung, umfassend oder bestehend aus:
(a) 5-Methyl-2-(propan-2-yl)cyclohexyl-N-ethyloxamat (Formel I) 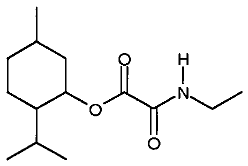 und
(b) ein, zwei, drei oder mehreren Polyolen, ausgewählt aus der Gruppe A bestehend aus verzweigten oder unverzweigten Alkandiolen und verzweigten oder unverzweigten Alkantriolen mit jeweils 3-12 Kohlenstoffatomen.

2. Kühlmischung nach Anspruch 1, wobei wenigstens ein Teil des 5-Methyl-2-(propan-2-yl)cyclohexyl-N-ethyloxamat in der (1R,2S,5R)-Konfiguration vorliegt (Formel II):

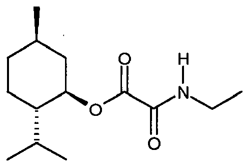

3. Kühlmischung nach Anspruch 2, wobei der Anteil 5-Methyl-2-(propan-2-yl)cyclohexyl-N-ethyloxamat, das in der (1R,2S,5R)-Konfiguration vorliegt, bezogen auf den Gesamtanteil an 5-Methyl-2-(propan-2-yl)cyclohexyl-N-ethyloxamat ≥45%, bevorzugt ≥70% und besonders bevorzugt ≥97% beträgt.

4. Kühlmischung nach einem der vorangehenden Ansprüche, wobei das Verhältnis der Stoffmenge von 5-Methyl-2-(propan-2-yl)cyclohexyl-N-ethyloxamat zu der Stoffmenge des Polyols oder der Polyole der Gruppe A gemeinsam 1:20 bis 1:0,1, bevorzugt 1:10 bis 1:0,5 und besonders bevorzugt 1:5 bis 1:1 beträgt.

5. Kühlmischung nach einem der vorangehenden Ansprüche, wobei ein oder das Polyol oder mehrere oder alle Polyole der Gruppe A ausgewählt ist oder sind aus der Gruppe bestehend aus den verzweigten oder unverzweigen Alkandiolen mit 3 bis 12 Kohlenstofatomen.

6. Kühlmischung nach einem der vorangehenden Ansprüche, wobei bei einem oder dem Alkanpolyol der Gruppe A oder mehreren oder allen Alkanpolyolen der Gruppe A beide oder wenigstens zwei der Hydroxygruppen zueinander vicinal stehen.

7. Kühlmischung nach einem der vorangehenden Ansprüche, wobei als Bestandteil (b) ausschliesslich ein oder mehrere verzweigte oder unverzweigte 1,2-Alkandiole mit 5-12 Kohlenstoffatomen eingesetzt werden.

8. Kühlmischung nach einem der vorangehenden Ansprüche, wobei der Bestandteil (b) n-1,2-Pentandiol umfasst oder daraus besteht.

9. Kühlmischung nach Anspruch 8, wobei das Verhältnis der Stoffmenge von 5-Methyl-2-(propan-2-yl)cyclohexy- N-ethyloxamat zu der Stoffmenge von n-1,2-Pentandiol 1:20 bis 1:0,1, bevorzugt 1:10 bis 1:1:0,5 und besonders bevorzugt 1:5 bis 1:1 beträgt.

10. Kühlmischung nach einem der vorangehenden Ansprüche, umfassend oder bestehend neben den Bestandteilen (a) und (b) aus *trans-* 4-tert.Butylcyclohexanol.

11. Kosmetische Zubereitung oder Hygieneartikel, umfassend eine Kühlmischung nach einem der vorangehenden Ansprüche.

12. Kosmetische Zubereitung nach Anspruch 11, ausgewählt aus der Gruppe bestehend aus Haarpflegemitteln und Hautpflegemitteln oder Hygieneartikel nach Anspruch 11, ausgewählt aus der Gruppe bestehend aus Damenbinden, Tampons und Windeln.

13. Verwendung eines, zweier, dreier oder mehrerer Polyole, ausgewählt aus der Gruppe A bestehend aus verzweigten oder unverzweigten Alkandiolen und verzweigten oder unverzweigten Alkantriolen mit jeweils 3-12 Kohlenstoffatomen zur Verstärkung der Kühlwirkung von 5-Methyl-2-(propan-2-yl)cyclohexyl N-ethyloxamat (Formel I) 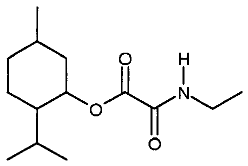 auf der Haut oder einer Schleimhaut.

14. Verfahren zum Erzeugen einer verstärkten Kühlwirkung von 5-Methyl-2-(propan-2-yl)cyclohexyl-N-ethyloxamat (Formel I) 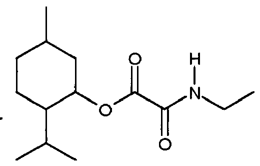 auf der Haut oder einer Schleimhaut, umfassend die folgenden Schritte:
a) Bereitstellen einer Kühlmischung nach einem der Ansprüche 1 bis 10 oder einer kosmetischen Zubereitung nach einem der Ansprüche 11 oder 12, wobei das Verhältnis der Stoffmenge von 5-Methyl-2-(propan-2-yl)cyclohexyl-N-ethyloxamat zu der Stoffmenge des Polyols oder der Polyole der Gruppe A gemeinsam 1:20 bis 1:0,1, bevorzugt 1:10 bis 1:0,5 und besonders bevorzugt 1:5 bis 1:1 beträgt, und
b) Kontaktieren der Kühlmischung oder der Kosmetischen Zubereitung mit Haut oder einer Schleimhaut.

15. Verfahren zum Erzeugen einer Kühlmischung nach einem der Ansprüche 1 bis 10 oder einer kosmetischen Zubereitung nach einem der Ansprüche 11 oder 12 umfassend die Schritte:
a) Bereitstellen von 5-Methyl-2-(propan-2-yl)cyclohexyl-N-ethyloxamat (Formel I) 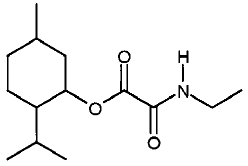
b) Bereitstellen eines, zweier, dreier oder mehrerer Polyole, ausgewählt aus der Gruppe A bestehend aus verzweigten oder unverzweigten Alkandiolen und verzweigten oder unverzweigten Alkantriolen mit jeweils 3-12 Kohlenstoffatomen und
c) Vermischen der in Schritt a) und b) bereitgestellten Komponenten.

## Claims

1. Cooling mixture comprising or consisting of:
(a) 5-methyl-2-(propane-2-yl)cyclohexyl-N-ethyloxamate (formula I) 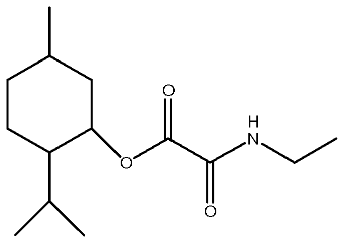 and
(b) one, two, three or more polyols, selected from the group A consisting of branched or unbranched alkanediols and branched or unbranched alkanetriols with each 3-12 carbon atoms.

2. The cooling mixture as claimed in Claim 1, wherein at least part of the 5-methyl-2-(propane-2-yl)cyclohexyl-N-ethyloxamate is present in a (1*R*,2*S*,5*R*) configuration (formula II):

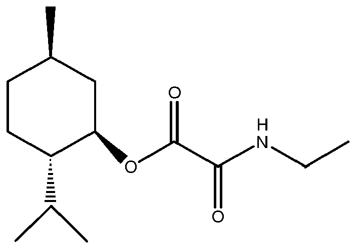

3. The cooling mixture as claimed in Claim 2, wherein the proportion of 5-methyl-2-(propane-2-yl)cyclohexyl-N-ethyloxamate present in the (1*R*,2*S*,5*R*) configuration, in relation to the overall proportion of 5-methyl-2-(propane-2-yl)cyclohexyl-N-ethyloxamate, is ≥ 45%, preferably ≥ 70% and particularly preferred ≥ 97%.

4. The cooling mixture according to any preceding claims, wherein the ratio of 5-methyl-2-(propane-2-yl)cyclohexyl-N-ethyloxamate to the amount of the polyols or the polyols of the group A in sum is 1:20 to 1:0,1, preferably 1:10 to 1:0,5, and particularly preferred 1:5 to 1:1.

5. The cooling mixture according to any preceding claims, wherein one or the polyol or several or all polyols of group A is selected or are selected from the group consisting of branched or unbranched alkandiols with 3 to 12 carbon atoms.

6. The cooling mixture according to any preceding claims, wherein at one or the alkanpolyol of group A or several or all alkanpolyols of the group A both or at least two of the hydroxyl groups are vicinal to one another.

7. The cooling mixture according to any preceding claims, wherein as an ingredient (b) one or more branched or unbranched 1,2-alkandiols with 5-12 carbon atoms are taken solely.

8. The cooling mixture according to any preceding claims, wherein the ingredient (b) comprises or consists of 1,2-pentandiol.

9. The cooling mixture as claimed in Claim 8, wherein the ratio of 5-methyl-2-(propane-2-yl)cyclohexyl-N-ethyloxamate to the amount of n-1,2-pentandiol is 1:20 to 1:0,1, preferably 1:10 to 1:0,5, and particularly preferred 1:5 to 1:1.

10. The cooling mixture according to any preceding claims, comprising or consisting besides ingredient (a) and (b) of trans-4-tert. butylcyclohexanol.

11. A cosmetic composition or sanitary article, comprising a cooling mixture as claimed in any of the preceding claims.

12. The cosmetic composition as claimed in Claim 11 selected from the group consisting of hair care products and skin care products or sanitary article according to claim 11, selected from the group consisting of sanitary napkins, tampons and diapers.

13. Use of one, two, three or more polyols selected from group A consisting of branched or unbranched alkandiols and branched or unbranched alkantriols with each 3-12 carbon atoms for enhancing the cooling effect of 5-methyl-2-(propane-2-yl)cyclohexyl-N-ethyloxamate (formula I) 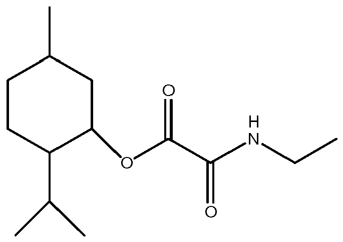 on the skin or a mucous membrane.

14. A method for generating an enhanced cooling effect of of 5-methyl-2-(propane-2-yl)cyclohexyl-N-ethyloxamate: 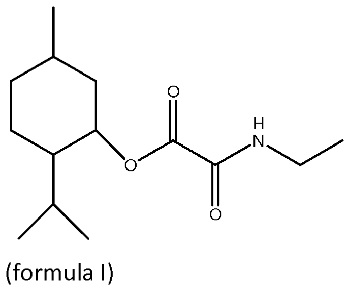 on the skin or a mucous membrane, comprising the following steps:
a) providing a cooling mixture according to any claims 1 to 10 or a cosmetic composition according to any claims 11 or 12, wherein the ratio between the amount of 5-methyl-2-(propane-2-yl)cyclohexyl-N-ethyloxamate to the amount of the polyols or the polyols of the group A is commonly 1:20 to 1:0,1, preferably 1:10 to 1:0,5, and particularly preferred 1:5 to 1:1; and
b) contacting the cooling mixture or the cosmetic composition with skin or a mucous membrane.

15. The method for generating a cooling mixture according to any claims 1 to 10 or a cosmetic composition according to any claims 11 or 12 comprising the steps:
a) providing of 5-methyl-2-(propane-2-yl)cyclohexyl-N-ethyloxamate (formula I) 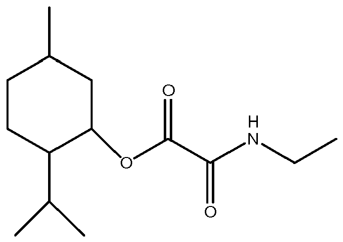
b) providing one, two, three or more polyols selected from group A consisting of branched or unbranched alkanediols and branched or unbranched alkanetriols with each 3-12 carbon atoms; and
c) mixing the components provided in steps a) and b).

## Revendications

1. Mélange refroidissant, comprenant ou constitué par :
(a) du N-éthyloxamate de 5-méthyl-2-(propan-2-yl)cyclohexyle (formule I) 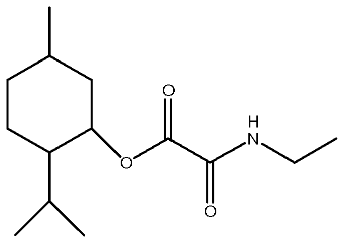 et
(b) un, deux, trois polyols ou plus, choisis dans le groupe A constitué par les alcanediols ramifiés ou non ramifiés et les alcanetriols ramifiés ou non ramifiés contenant chacun 3 à 12 atomes de carbone.

2. Mélange refroidissant selon la revendication 1, dans lequel au moins une partie du N-éthyloxamate de 5-méthyl-2-(propan-2-yl)cyclohexyle se présente selon la configuration (1R,2S,5R) (formule II) :

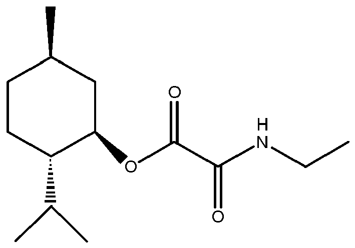

3. Mélange refroidissant selon la revendication 2, dans lequel la proportion de N-éthyloxamate de 5-méthyl-2-(propan-2-yl)cyclohexyle qui se présente selon la configuration (1R,2S,5R), par rapport à la proportion totale de N-éthyloxamate de 5-méthyl-2-(propan-2-yl)cyclohexyle, est ≥ 45 %, de préférence ≥ 70 % et de manière particulièrement préférée ≥ 97 %.

4. Mélange refroidissant selon l'une quelconque des revendications précédentes, dans lequel le rapport entre la quantité de matière de N-éthyloxamate de 5-méthyl-2-(propan-2-yl)cyclohexyle et la quantité de matière du polyol ou des polyols du groupe A ensemble est de 1:20 à 1:0,1, de préférence de 1:10 à 1:0,5 et de manière particulièrement préférée de 1:5 à 1:1.

5. Mélange refroidissant selon l'une quelconque des revendications précédentes, dans lequel un ou le polyol ou plusieurs ou tous les polyols du groupe A sont choisis dans le groupe constitué par les alcanediols ramifiés ou non ramifiés de 3 à 12 atomes de carbone.

6. Mélange refroidissant selon l'une quelconque des revendications précédentes, dans lequel, pour un ou l'alcanepolyol du groupe A ou plusieurs ou tous les alcanepolyols du groupe A, les deux ou au moins deux des groupes hydroxy sont en position vicinale l'un par rapport à l'autre.

7. Mélange refroidissant selon l'une quelconque des revendications précédentes, dans lequel exclusivement un ou plusieurs 1,2-alcanediols ramifiés ou non ramifiés de 5 à 12 atomes de carbone sont utilisés en tant que constituant (b).

8. Mélange refroidissant selon l'une quelconque des revendications précédentes, dans lequel le constituant (b) comprend du n-1,2-pentanediol ou en est constitué.

9. Mélange refroidissant selon la revendication 8, dans lequel le rapport entre la quantité de matière de N-éthyloxamate de 5-méthyl-2-(propan-2-yl)cyclohexyle et la quantité de matière de n-1,2-pentanediol est de 1:20 à 1:0,1, de préférence de 1:10 à 1:0,5 et de manière particulièrement préférée de 1:5 à 1:1.

10. Mélange refroidissant selon l'une quelconque des revendications précédentes, comprenant ou constitué par, en plus des constituants (a) et (b), du *trans*-4-tert.-butylcyclohexanol.

11. Préparation cosmétique ou article d'hygiène, comprenant un mélange refroidissant selon l'une quelconque des revendications précédentes.

12. Préparation cosmétique selon la revendication 11, choisie dans le groupe constitué par les produits capillaires et les produits pour la peau, ou article d'hygiène selon la revendication 11, choisi dans le groupe constitué par les serviettes hygiéniques, les tampons et les couches.

13. Utilisation d'un, de deux, de trois polyols ou plus, choisis dans le groupe A constitué par les alcanediols ramifiés ou non ramifiés et les alcanetriols ramifiés ou non ramifiés contenant chacun 3 à 12 atomes de carbone, pour renforcer l'effet refroidissant du N-éthyloxamate de 5-méthyl-2-(propan-2-yl)cyclohexyle (formule I) 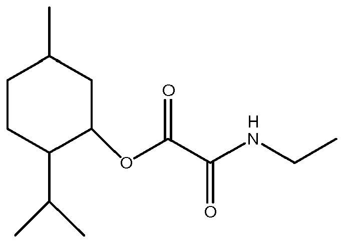 sur la peau ou une muqueuse.

14. Procédé de génération d'un effet refroidissant renforcé du N-éthyloxamate de 5-méthyl-2-(propan-2-yl)cyclohexyle (formule I) 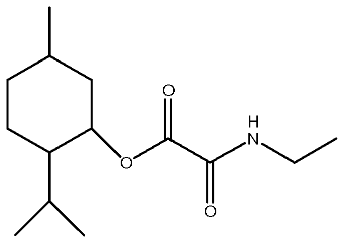 sur la peau ou une muqueuse, comprenant les étapes suivantes :
a) la préparation d'un mélange refroidissant selon l'une quelconque des revendications 1 à 10 ou d'une préparation cosmétique selon l'une quelconque des revendications 11 ou 12, le rapport entre la quantité de matière de N-éthyloxamate de 5-méthyl-2-(propan-2-yl)cyclohexyle et la quantité de matière du polyol ou des polyols du groupe A ensemble étant de 1:20 à 1:0,1, de préférence de 1:10 à 1:0,5 et de manière particulièrement préférée de 1:5 à 1:1, et
b) la mise en contact du mélange refroidissant ou de la préparation cosmétique avec la peau ou une muqueuse.

15. Procédé de formation d'un mélange refroidissant selon l'une quelconque des revendications 1 à 10 ou d'une préparation cosmétique selon l'une quelconque des revendications 11 ou 12, comprenant les étapes suivantes :
a) la préparation de N-éthyloxamate de 5-méthyl-2-(propan-2-yl)cyclohexyle (formule I) 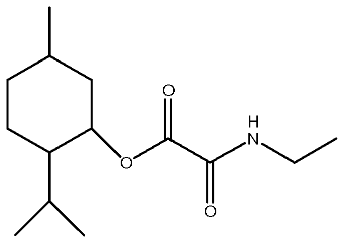
b) la préparation d'un, de deux, de trois polyols ou plus, choisis dans le groupe A constitué par les alcanediols ramifiés ou non ramifiés et les alcanetriols ramifiés ou non ramifiés contenant chacun 3 à 12 atomes de carbone, et
c) le mélange des composants préparés à l'étape a) et b).
